# EUROPEAN PATENT SPECIFICATION

(11) **EP 1 909 830 B1**
(45) Date of publication and mention of the grant of the patent: **28.09.2011**
(21) Application number: 06795518.7
(22) Date of filing: 02.08.2006
(51) Int. Cl.: A61K 39/39

(54) **REDUCING INTERFERENCE BETWEEN OIL-CONTAINING ADJUVANTS AND SURFACTANT-CONTAINING ANTIGENS**
REDUZIERUNG DER INTERFERENZ ZWISCHEN ÖLHALTIGEN ADJUVANZIEN UND SURFACTANTHALTIGEN ANTIGENEN
REDUCTION DES INTERACTIONS ENTRE ADJUVANTS HUILEUX ET ANTIGENES TENSIOACTIFS

(30) Priority: 02.08.2005 GB 0515906; 04.11.2005 GB 0522599; 24.11.2005 GB 0523923
(43) Date of publication of application: 16.04.2008
(73) Proprietor: Novartis Vaccines and Diagnostics S.r.l., 53100 Siena (SI) (IT)
(72) Inventor: CONTORNI, Mario, Novartis Vaccines&Diagnostics SRL, I-53100 Siena (IT)
(74) Representative: Marshall, Cameron John
(86) International application number: PCT/IB2006/002581
(87) International publication number: WO 2007/015167

(56) References cited:
- EP-A2- 0 781 559
- WO-A-98/05355
- WO-A2-00/03744
- WO-A2-98/43670
- US-A- 3 435 112
- DATABASE CA [Online] CHEMICAL ABSTRACTS SERVICE, COLUMBUS, OHIO, US; HASHIMOTO, SATORU ET AL: "Ether-type surfactant -free oil adjuvants and animal vaccines containing the adjuvants" XP002418606 retrieved from STN Database accession no. 2001:403420 & JP 2001 151699 A (NIHON SURFACTANTS INDUSTRY CO., LTD., JAPAN; MICROBIOCHEMICAL RESEARCH) 5 June 2001 (2001-06-05)
- TRAQUINA P ET AL: "MF59 ADJUVANT ENHANCES THE ANTIBODY RESPONSE TO RECOMBINANT HEPATITIS B SURFACE ANTIGEN VACCINE IN PRIMATES" JOURNAL OF INFECTIOUS DISEASES, CHICAGO, IL, US, vol. 174, no. 6, 1996, pages 1168-1175, XP000971687 ISSN: 0022-1899

## Description

### TECHNICAL FIELD

This invention is in the field of manufacturing adjuvanted vaccines. In particular, it concerns the use of fatty adjuvants during the manufacture of vaccines based on surfactant-containing antigens.

### BACKGROUND ART

The use of adjuvants to enhance immune responses against vaccine antigens is well known (*e.g*. see references 1 and 2). For many years the only adjuvants approved for human use were aluminium 3 salts, but vaccines containing the MF59 adjuvant and the RC-529 adjuvant have been approved in various countries, including Italy (in Chiron's FLUAD™ product) and Argentina (in Berna Biotech's SUPERVAX™ product). Further adjuvants in late-stage human experimental use include mixtures of cholesterol and saponins, ISCOMs, MPL™, AS04, virosomes, SBA4, *etc*. MPL™, or 3-O-deacylated monophosphoryl lipid A, has been used with HBsAg e.g see WO 98/43670.

A common feature of some alternatives to aluminium salts is the presence of a fatty component. For example, the MF59 adjuvant includes squalene (an oil), and MPL™ contains a deacylated form of monophosphoryl lipid A having multiple fatty acid chains attached to a di-glucosamine backbone.

The invention concerns the avoidance of interference that can occur between these fatty adjuvants and antigens that include a surfactant component.

### SUMMARY OF THE INVENTION

The inventor has found that fatty adjuvants in vaccine compositions can be incompatible with antigens that include a surfactant component. In many situations, however, it remains desirable to combine a fatty adjuvant and an antigen/surfactant mixture, and it is an object of the invention to provide ways of avoiding the difficulties that can arise in doing so. It is a further object to provide processes for combining adjuvants and antigens, in which incompatibility is avoided.

To avoid these incompatibility problems when using antigens that are typically purified by using surfactants, one approach would be to use a process for preparing an immunogenic composition, wherein: (a) the composition comprises an antigen and a fatty adjuvant; and (b) the antigen is purified substantially in the absence of surfactant. By purifying the antigen by an alternative route that avoids the use of surfactant, any incompatibility with fatty adjuvants would be overcome.

For clinical, historical or regulatory reasons, however, it may not be possible to purify an antigen without the use of surfactants, or to totally remove surfactants from vaccines. In this situation, the invention avoids interference by reducing the ratio of oil to surfactant in the composition. A typical MF59/HBsAg vaccine contains a huge excess of oil relative to surfactant, with an oil:surfactant ratio of over 4000:1 (by weight). The invention aims to minimise the amount of fatty adjuvant in a composition and uses at most a 1000-fold weight excess of oil. Thus the invention provides a process for preparing an immunogenic composition, comprising the steps of combining (i) a viral surface antigen component that includes a surfactant and (ii) a fatty adjuvant component, to give a composition in which the weight ratio of the fatty adjuvant to the surfactant is less than 1000:1.

### The fatty adjuvant

The invention utilises fatty adjuvant *i.e*. an adjuvant component that includes a fatty molecule. Typical fatty adjuvants comprise a metabolisable oil, a fatty acid and/or a molecule comprising a fatty acid moiety.

Two preferred fatty adjuvants are the adjuvants known as 'MF59' and 'MPL'. 'MF59' is a fatty adjuvant because it contains squalene, which is a metabolisable oil. 'MPL' is a fatty adjuvant because it contains a disaccharide substituted with multiple fatty acid chains. As well as MF59, other oil-in-water emulsion adjuvants can also be used.

Thus preferred compositions of the invention include: (a) an oil-in-water emulsion, such as a sub-micron oil-in-water emulsion comprising squalene and polyoxyethylene sorbitan monooleate (and, optionally, sorbitan trioleate); and/or (b) a 3-O-deacylated monophosphoryl lipid A. Polyoxyethylene sorbitan monooleate is also known as polysorbate 80.

In addition to 'MF59' and 'MPL', other fatty adjuvants that can be used with the invention include, but are not limited to: glucosaminide phosphate derivatives; N-acyl-pseudodipeptides; the soluble adjuvant derived from *Escherichia coli* lipid A known as 'OM-174'; compounds containing lipids linked to a phosphate-containing acyclic backbone; and acyclic synthetic lipid A analogs.

Where an oil-in-water emulsion adjuvant such as MF59 is used, it is typically added to an equal volume of an aqueous antigen composition, such that the emulsion is 50% by volume of the total composition. Where a 3D-MPL adjuvant is used, a typical dose is between 25µg/ml and 200µg/ml *e.g.* in the range 50-150µg/ml, 75-125µg/ml, 90-110µg/ml, or about 100µg/ml. It is usual to administer between 25-75µg of 3D-MPL per dose *e.g*. between 45-55µg, or about 50µg 3D-MPL per dose. An emulsion adjuvant such as MF59 will typically be provided in a separate container from the antigen, for extemporaneous mixing at the time of use, or it can be provided already admixed with the antigen. A 3D-MPL adjuvant will typically be already admixed with the antigen before distribution to end users.

### MF59

The 'MF59' adjuvant is an oil-in-water emulsion formed from squalene, Tween 80 (polyoxyethylene sorbitan monooleate) and Span 85 (sorbitan trioleate). The emulsion is microfluidised to give an emulsion with a submicron droplet size. Preparation of MF59 was originally described in reference 3, and the product has been manufactured and sold by Chiron Corporation. Further details can be found in Chapter 10 of ref. 2, Chapter 12 of ref. 1 and in references 4 to 6. The composition of MF59 by volume is 5% squalene, 0.5% polysorbate 80 and 0.5% Span 85. In weight terms, these ratios become 4.3% squalene, 0.5% polysorbate 80 and 0.48% Span 85. MF59's own surfactant content is not taken into account when calculating the ratio of oil:surfactant, as the ratio is based on the surfactant content of the antigen.

The MF59 emulsion advantageously includes citrate ions *e.g*. 10mM sodium citrate buffer.

### Other oil-in-water emulsions

As an alternative to the MF59 adjuvant, other oil-in-water emulsions can be used. Adjuvant emulsions typically include at least one oil and at least one surfactant, with the oil(s) and surfactant(s) being biodegradable (metabolisable) and biocompatible. The oil droplets in the emulsion are generally less than 5µm in diameter, typically with a sub-micron diameter. Small droplet sizes can be achieved with a microfluidiser to provide stable emulsions. Droplets with a size less than 220nm are preferred as they can be subjected to filter sterilization.

The emulsions can include oils such as those from an animal (such as fish) or vegetable source, rather than mineral oils. Sources for vegetable oils include nuts, seeds and grains. Peanut oil, soybean oil, coconut oil, and olive oil, the most commonly available, exemplify the nut oils. Jojoba oil can be used *e.g*. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X-100. Mixtures of surfactants can be used *e.g*. Tween 80/Span 85 mixtures, or Tween80/Triton-X100 mixtures.

Oil-in-water emulsion adjuvants useful with the invention include, but are not limited to:
- An emulsion of squalene, a tocopherol, and Tween 80. The emulsion may include phosphate buffered saline. It may also include Span 85 (*e.g*. at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm.
- An emulsion of squalene, a tocopherol, and a Triton detergent (*e.g*. Triton X-100).
- An emulsion of squalane, polysorbate 80 and poloxamer 401 ("Pluronic™ L121"). The • emulsion can be formulated in phosphate buffered saline, pH 7.4. This emulsion is a useful delivery vehicle for muramyl dipeptides, and has been used with threonyl-MDP in the "SAF-1" adjuvant [7] (0.05-1% Thr-MDP, 5% squalane, 2.5% Pluronic L121 and 0.2% polysorbate 80). It can also be used without the Thr-MDP, as in the "AF" adjuvant [8] (5% squalane, 1.25% Pluronic L121 and 0.2% polysorbate 80). Microfluidisation is preferred.
- An emulsion having from 0.5-50% of an oil, 0.1-10% of a phospholipid, and 0.05-5% of a non-ionic surfactant. As described in reference 9, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 10, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin *(e.g.* QuilA or QS21) and a sterol *(e.g.* a cholesterol) are associated as helical micelles [11].

The emulsions are preferably mixed with antigen extemporaneously, at the time of delivery. Thus the adjuvant and antigen are typically kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e*.*g*. between 5:1 and 1:5) but is generally about 1:1.

There a composition includes a tocopherol, any of the α, β, γ, δ, ε or ζ tocopherols can be used, but α-tocopherols are preferred. The tocopherol can take several forms *e.g*. different salts and/or isomers. Salts include organic salts, such as succinate, acetate, nicotinate, *etc.* D-α-tocopherol and DL-α-tocopherol can both be used. A preferred α-tocopherol is DL-α-tocopherol, and the preferred salt of this tocopherol is the succinate.

### 3D-MPL

3-O-deacylated monophosphoryl lipid A (3D-MPL; also referred to as 3 de-O-acylated monophosphoryl lipid A or 3-O-desacyl-4'-monophosphoryl lipid A) is a known adjuvant. The name indicates that position 3 of the reducing end glucosamine in monophosphoryl lipid A is de-acylated. 3D-MPL has been prepared from a heptoseless mutant of *Salmonella minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF. Preparation of 3D-MPL was originally described in reference 12, and the product has been manufactured and sold by Corixa Corporation under the trade name 'MPL'. Further details can be found in references 13 to 16.

3D-MPL can take the form of a mixture of related molecules, varying by their acylation (*e.g*. having 3, 4, 5 or 6 acyl chains, which may be of different lengths). The two glucosamine (also known as 2-deoxy-2-amino-glucose) monosaccharides are N-acylated at their 2-position carbons (*i.e*. at positions 2 and 2'), and there is also O-acylation at the 3' position. The group attached to carbon 2 has formula -NH-CO-CH₂-CR¹R¹. The group attached to carbon 2' has formula -NH-CO-CH₂-CR²R^{2'} . The group attached to carbon 3' has formula -O-CO-CH₂-CR³R^{3'}. A representative structure is:

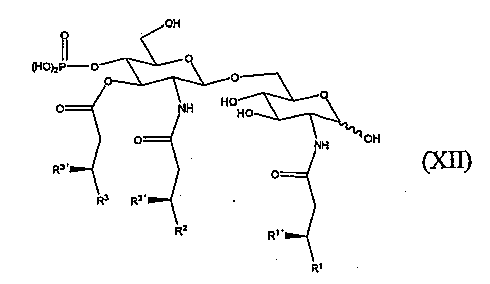

Groups R¹, R² and R³ are each independently -(CH₂)ₙ-CH₃. The value of *n* is preferably between 8 and 16, more preferably between 9 and 12, and is most preferably 10.

Groups R^{1'}, R^{2'} and R^{3'} can each independently be: (a) -H; (b) -OH; or (c) -O-CO-R⁴,where R⁴ is either -H or -(CH₂)ₘ-CH₃, wherein the value of *m* is preferably between 8 and 16, and is more preferably 10, 12 or 14. At the 2 position, *m* is preferably 14. At the 2' position, *m* is preferably 10. At the 3' position, *m* is preferably 12. Groups R^{1'}, R^{2'} and R^{3'} are thus preferably -O-acyl groups from dodecanoic acid, tetradecanoic acid or hexadecanoic acid.

When all of R^{1'}, R^{2'} and R^{3'} are -H then the 3D-MPL has only 3 acyl chains (one on each of positions 2, 2' and 3'). When only two of R^{1'}, R^{2'} and R^{3'} are -H then the 3D-MPL can have 4 acyl chains. When only one of R^{1'}, R^{2'} and R^{3'} is -H then the 3D-MPL can have 5 acyl chains. When none of R^{1'}, R^{2'} and R^{3'} is -H then the 3D-MPL can have 6 acyl chains. The 3D-MPL adjuvant used according to the invention can be a mixture of these forms, with from 3 to 6 acyl chains, but it is preferred to include 3D-MPL with 6 acyl chains in the mixture, and in particular to ensure that the hexaacyl chain form makes up at least 10% by weight of the total 3D-MPL *e.g*. ≥20%, ≥30%, ≥40%, ≥50% or more. 3D-MPL with 6 acyl chains has been found to be the most adjuvant-active form.

Thus the most preferred form of 3D-MPL for inclusion in compositions of the invention is:

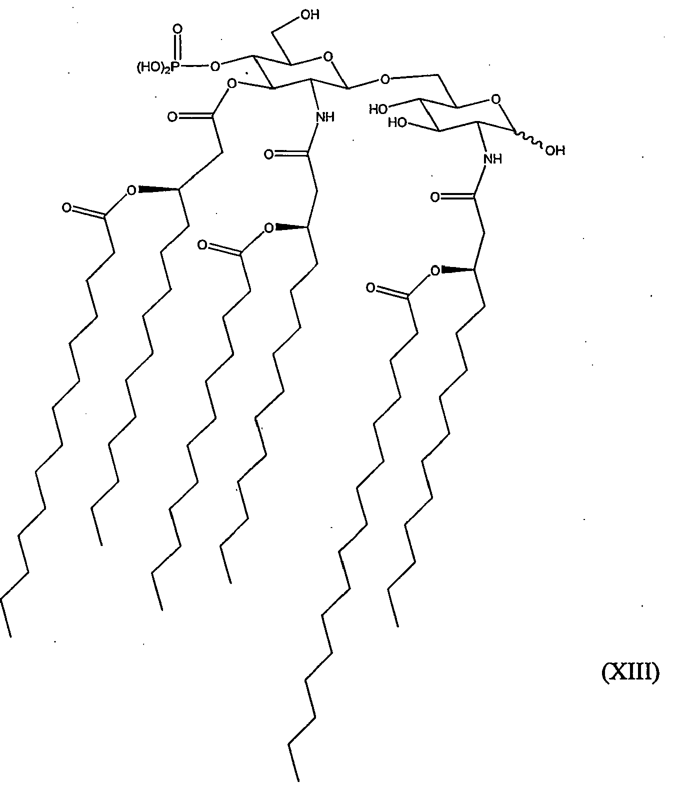

Where 3D-MPL is used in the form of a mixture then references to amounts or concentrations of 3D-MPL in compositions of the invention refer to the combined 3D-MPL species in the mixture.

In aqueous conditions, 3D-MPL can form micellar aggregates or particles with different sizes *e.g*. with a diameter <150nm or >500nm. Either or both of these can be used with the invention, and the better particles can be selected by routine assay. Smaller particles (*e.g*. small enough to give a clear aqueous suspension of 3D-MPL) are preferred for use according to the invention because of their superior activity [17]. Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilisation. Particle diameter can be assessed by the routine technique of dynamic light scattering, which reveals a mean particle diameter. Where a particle is said to have a diameter of x nm, there will generally be a distribution of particles about this mean, but at least 50% by number (*e.g*. ≥60%, ≥70%. ≥80%, ≥90%, or more) of the particles will have a diameter within the range *x*±25%.

The 3D-MPL can be used on its own as an adjuvant, or in combination with one or more further adjuvant compounds. For example, it is known to use 3D-MPL in combination with the QS21 saponin [18], with QS21 and an oil-in-water emulsion [19], with aluminium phosphate. [20], with aluminium hydroxide [21], or with saponins & interleukin-12 [22].

A preferred adjuvant mixture, particularly for use with HBsAg, comprises a mixture of 3D-MPL and an aluminium salt, preferably aluminium phosphate. Advantageously, the 3D-MPL is adsorbed onto the aluminium salt. Preferably at least 50% (by weight) of the 3D-MPL is adsorbed *e*.*g*. ≥60%, ≥70%, ≥80%, ≥90%, ≥95%, ≥98% or more.

Aluminium adjuvants currently in use are typically referred to either as "aluminium hydroxide" or as "aluminium phosphate" adjuvants. These are names of convenience, however, as neither is a precise description of the actual chemical compound which is present (*e.g*. see chapter 9 of reference 2). For combination with a fatty adjuvant such as 3D-MPL, the invention can use any of the "hydroxide" or "phosphate" salts that are in general use as adjuvants.

The adjuvants known as "aluminium hydroxide" are typically aluminium oxyhydroxide salts, which are usually at least partially crystalline. Aluminium oxyhydroxide, which can be represented by the formula AIO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹ (chapter 9 of ref. 2).

The adjuvants known as "aluminium phosphate" are typically aluminium hydroxyphosphates, often also containing a small amount of sulfate. They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 0.99. Hydroxyphosphates can be distinguished from strict AIPO₄ by the presence of hydroxyl groups. For example, an IR spectrum band at 3164cm⁻¹ (*e.g*. when heated to 200°C) indicates the presence of structural hydroxyls (chapter 9 of ref. 2).

The PO₄/Al³⁺ molar ratio of an aluminium phosphate adjuvant will generally be between 0.3 and 1.2, preferably between 0.8 and 1.2, and more preferably 0.95±0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg A1³⁺/ml. The aluminium phosphate will generally be particulate. Typical diameters of the particles are in the range 0.5-20µm (*e.g*. about 5-10µm) after any antigen adsorption.

The PZC of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g*. about 5.7.

An aluminium phosphate solution used to prepare a composition of the invention may contain a buffer (*e.g*. a phosphate or a histidine or a Tris buffer), but this is not always necessary. The aluminium phosphate solution is preferably sterile and pyrogen-free. The aluminium phosphate solution may include free aqueous phosphate ions *e.g*. present at a concentration between 1.0 and 20 mM, preferably between 5 and 15 mM, and more preferably about 10 mM. The aluminium phosphate solution may also comprise sodium chloride. The concentration of sodium chloride is preferably in the range of 0.1 to 100 mg/ml (*e.g*. 0.5-50 mg/ml, 1-20 mg/ml, 2-10 mg/ml) and is more preferably about 3±1 mg/ml. The presence of NaCl facilitates the correct measurement of pH prior to adsorption of antigens.

The aluminium phosphate is preferably used in the form of an aqueous solution to which 3D-MPL (and, optionally, an antigen) is added (NB: it is standard to refer to aqueous aluminium phosphate as a "solution" although, on a strict physicochemical view, the salt is insoluble and forms a suspension). It is preferred to dilute the aluminium phosphate to the required concentration and to ensure a homogenous solution before the addition of the 3D-MPL and/or the antigen.

### N-acyl-pseudodipeptides

A N-acyl-pseudodipeptide adjuvant preferably includes a hydroxyl group that is esterified by an acid group in the neutral or charged form. The acyl groups give the pseudodipeptides a fatty character. The adjuvant preferably has formula (I): 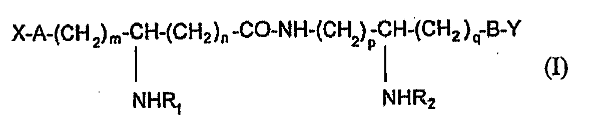 where:
- R₁ is an acyl group derived from a saturated or unsaturated, straight or branched chain-carboxylic acid having from 2 to 24 carbon atoms, the carboxylic acid being unsubstituted or substituted with one or more substituents selected from the group consisting of hydroxyl, alkyl, alkoxy, acyloxy, amino, acylamino, acylthio and ((C₁₋₂₄)alkyl)thio groups;
- R₂ is an acyl group derived from a saturated or unsaturated, straight or branched chain-carboxylic acid having from 2 to 24 carbon atoms, the carboxylic acid being unsubstituted or substituted with one or more substituents selected from the group consisting of hydroxyl, alkyl, alkoxy, acyloxy, amino, acylamino, acylthio and ((C₁₋₂₄)alkyl)thio groups;
- X is a hydrogen atom or an acid group either in neutral or charged form;
- Y is a hydrogen atom or an acid group either in neutral or charged form;
- A is an oxygen atom, a sulfur atom or an imino group -NH-;
- B is an oxygen atom, a sulfur atom or an imino group -NH-;
- in is 1, 2, 3, 4, 5, 6, 7, 8, 9 or 10;
- n is 0, 1, 2, 3, 4, 5, 6, 7, 8, 9 or 10;
- p is 1, 2, 3, 4, 5, 6, 7, 8, 9 or 10; and
- is 1, 2, 3, 4, 5, 6, 7, 8, 9 or 10,
provided that at least one of substituents X or Y designates an acid group in neutral or ionic form.

The adjuvant may be used either in acid or salt form, with an organic or mineral base. '

salt forming bases intended for therapeutic use mainly include alkaline metal bases such as sodium, potassium or lithium hydroxides, ammonium salts; alkali earth metal bases such as calcium or strontium hydroxide and magnesium salts; ferrous metal salts and the like; organic bases such as those derived from primary, secondary, tertiary amines such as methylamine, diethylamine, monoethanolamine, diethanolamine, benzylamine, N-methylbenzylamine, veratrylamine, trimethoxybenzylamine; basic amino acids such as lysine and ornithine or amino sugars. Examples of bases not intended for therapeutic use are brucine, strychnine, agmatine, homarine, glucosamine, N-methylglucosamine or N-methylmorpholine.

Preferred substituents X and Y are selected from:
carboxy [(C₁₋₅)alkyl]
- CH-[(CH₂)ᵣCOOH] [(CH₂)ₛCOOH], where: r is 0,1, 2, 3, 4 or 5; s is 0, 1, 2, 3, 4 or 5
- phosphono [(C₁₋₅)alkyl]
- dihydroxyphosphoryloxy [(C₁₋₅)alkyl]
- dimethoxyphosphoryl
- phosphono
- hydroxysulfonyl
- hydroxysulfonyl[(C₁₋₅)alkyl]
- hydroxysulfonyloxy [(C₁₋₅)alkyl]

Where substituents X and/or Y designate an acid group in neutral form, reference is made to the free carboxylic, sulfonic or phosphoric form. Where the acid group is in charged form, reference is made to the carboxylic, sulfonic or phosphoric salt form, namely by addition of an organic or mineral base, preferably one intended for therapeutic use. Similar considerations apply where X and/or Y designate a carboxylalkyl, alkenylbiscarboxylic, hydroxysulfonyl, hydroxysulfonylalkyl, hydroxysulfonyl-oxyalkyl, phosphonoalkyl, or a phosphoryl-oxyakyl group.

When m=1 and n=0, the adjuvant derives from serine. Where m=2 and n=0, the adjuvant derives from homoserine. If m=3 and n=0, reference is made to a pentahomoserine compound. If m=4 and n=0, reference is made to a hexahomoserine compound.

Where p=3 and q=1, the product of interest may be a citrulline, ornithine or arginine compound. Where p=4 and q=1, reference is made to a homoarginine or lysine compound.

R₁ and R₂ include saturated or unsaturated, branched or straight chain, acyl derivatives having a variable size chain, of distinct or identical nature, which can have one or more substituents selected from the group consisting of alkyl, amino, acylamino, hydroxyl, alkoxy, acyloxy, acylthio and alkylthio groups.

Examples of such acylated, substituted derivatives are ricinoleyl, 1,2-hydroxystearoyl, 2-hydroxy-3-methyl butyroyl, 3-hydroxy-2-aminopentanoyl, palmitoyl, elaidyl; eleostearoyl, arachidoyl, arachidonyl, gadoleyl, behenyl, erucyl, 8-methyldecanoyl, 9-methyldecanoyl, docosohexaenoyl or eicosapentaneoyl radicals.

Preferred adjuvants have formula (Ia), where A and B are both oxygen atoms:

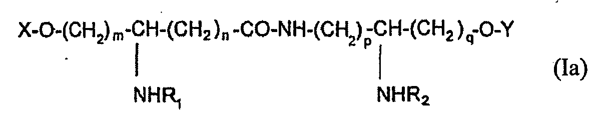

In formula (Ia), X and Y are preferably either a hydrogen atom or a phosphono group.

Suitable adjuvants of formula (Ia) include:
- 3-(3-dodecanoyloxytetradecanoylamino) 9-(3-hydroxytetradecanoylamino) 4-oxo-5-azadecan-1,10-diol 1 and/or 10- dihydrogenphosphate and its addition salts formed with an organic or a mineral base,
- 3-(3-dodecanoyloxy-tetradecanoylamino) 9-(3-hydroxytetradecanoylamino) 4-oxo-5-azadecan-1,10-diol 1,10-bis(dihydrogenphosphate) and its addition salts formed with an organic or a mineral base,
- 3-(3-hydroxytetradecanoylamino) 9-(3-dodecanoyloxytetradecanoylamino) 4-oxo-5-azadecan-1,10-diol 1,10-bis(dihydrogenphosphate) and its addition salts formed with an organic or a mineral base,
- 3-(3-dodecanoyloxytetradecanoylamino) 9-(3-hydroxytetradecanoylamino) 4-oxo-5-azadecan-1,10-diol 1-dihydrogenphosphate and its addition salts formed with an organic or a mineral base,
- 3-(3-hydroxytetradecanoylamino) 9-(3-dodecanoyloxytetradecanoylamino) 4-oxo-5-azadecan-1,10-diol 1-dihydrogenphosphate and its addition salts formed with an organic or a mineral base
- 3-(3-hydroxytetradecanoylamino) 9-(3-dodecanoyloxytetradecanoylamino) 4-oxo-5-azadecane-1,10-diol 10-dihydrogenphosphate and its addition salts formed with an organic or a mineral base.

Methods for synthesising adjuvants of formula (I) are disclosed in detail in reference 23, and include methods comprising the steps of: protecting amine functional groups in positions (q+1) and ω of a diamino acid by blocking reagents which readily undergo acidolysis and hydrogolysis, respectively; reacting the free carboxylic functional group with a reducing agent to yield a corresponding alcohol; de-protecting the amine functional group in position (q+1) and then acylating by means of a carboxylic acid functional derivative of formula R₂OH, and subsequently freeing the terminal amine functional group by hydrogenolysis to yield a diamino alcohol of general formula II: 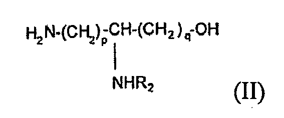 which amino alcohol is condensed in presence of a peptide condensing agent in an inert solvent, together with a ω-hydroxy, ω-amino or ω-thio amino acid compound of general formula III: 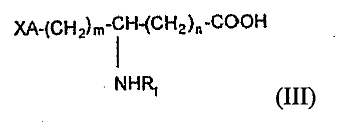 to provide a dipeptide compound of formula (IV): 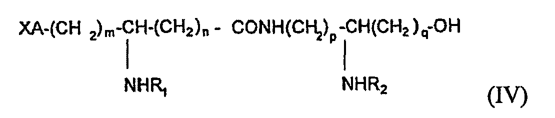 the terminal free alcohol functional group of which can be, if necessary, protected (*e.g*. by an alkyl or acyl group, or any other suitable protecting group) or otherwise substituted, if needed, in presence of a coupling agent. The protected alcohol may be subjected to a catalytic hydrogenation or other de-protection treatment in order to obtain the derivative of general formula I.

Methods for synthesising adjuvants of formula (II) are also disclosed in detail in reference 23, and include methods comprising the steps of: protecting amine functional groups in positions (q+1) and ω of a diamino acid of formula H₂N(CH₂)ₚCHNH₂(CH₂)_{q+1}COOH by protecting reagents which readily undergo acidolysis and hydrogenolysis, respectively; reacting the free carboxylic functional group with a reducing agent to yield a corresponding alcohol, de-protecting the amine functional group in position (q+1) and then acylating by means of a carboxylic acid functional derivative of formula R₂OH then de-protecting the terminal amine functional group by hydrogenolysis to obtain an amino alcohol of general (formula II; which amino alcohol is condensed in presence of a peptide condensing agent in an inert solvent, together with an ω-hydroxy amino acid functional derivative of general formula IIIa: 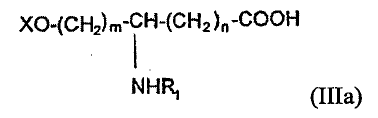 where X is dialkyloxy- or diaryloxy- phosphoryl radical of formula (RO)₂P-O to give a peptide-like compound of formula IVa: 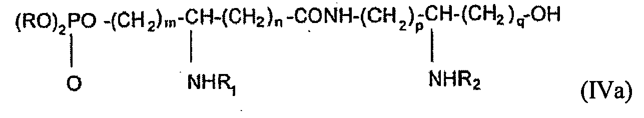 where R is a radical which readily undergoes hydrogenolysis, the other alcohol functional group of which can be, if desired, phosphorylated by a phosphorylating agent in presence of a coupling agent, if needed. The protected alcohol may be subjected to a catalytic hydrogenation on one hand in order to de-protect the alcohol functional group optionally present on acyl group R₂ and on the other, free the phosphate functional group and then de-protect through hydrogenolysis the second optionally present phosphate functional group, in order to obtain the derivative of general formula V: 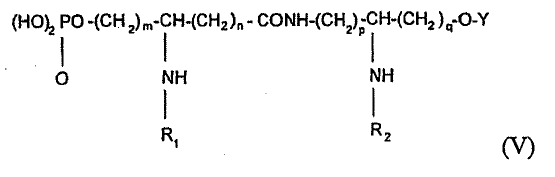 and optionally performing the further step of salt formation by means of an organic or mineral base.

Stereochemistry of chiral centres of acylamino groups is determined by initially used amino acid configuration whereas stereochemistry of acylamino groups depends on initially used fatty acid configuration. One can start from a diamino acid having L or D configuration or of a racemic nature. One can start from a hydroxylated amino acid of L, D configuration or of a racemic mixture. All such stereoisomers or diastereoisomers are included in the scope of the invention.

Particularly preferred adjuvants, and notably mono- and bis-phosphorylated compounds, are those which are known as "OM-294-MP" (formula VI) and "OM-294-DP" (formula VII) [23]:

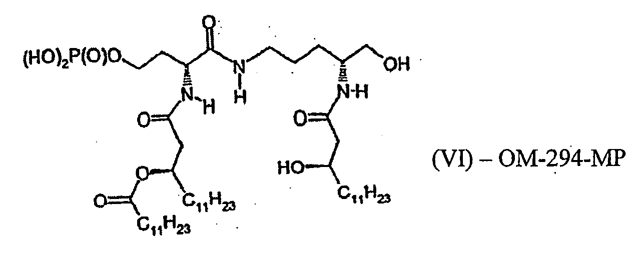

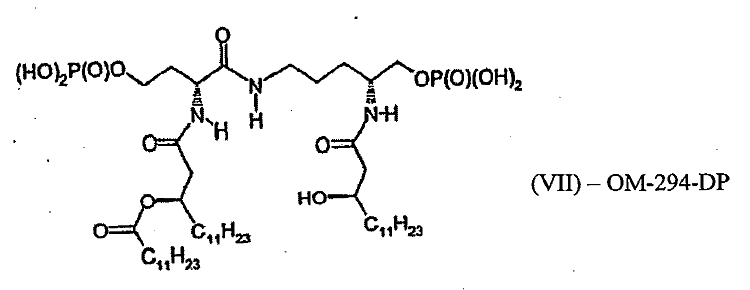

### OM-174

OM-174 can be obtained through chemical synthesis, and retains a triacyl motif from lipid A. It is described in more detail in references 24-26. Reference 26 gives the formula of OM-174 as:

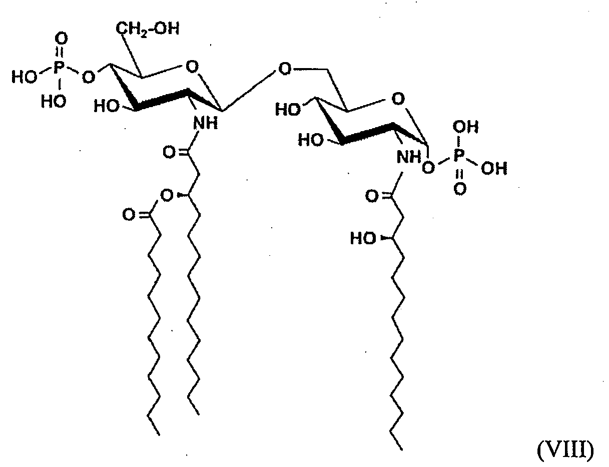

Thus OM-174 has a 1,4'-biphosphorylated (β(1→6)-linked diglucosamine backbone, as found in natural lipid A. OM-174 may be present in the form of aggregates with a micellar H_{I} structure *i.e*. in which the lipid molecules are packed with their backbone on a cylindrical surface with the acyl chains directed inwards, with the cylinders themselves being hexagonally packed. A cubic phase may be absent. A H_{II} phase may also be absent.

### Glucosaminide phosphate derivatives

Aminoalkyl glucosaminide phosphate (AGP) derivatives (*e.g*. RC-529 [27,28]) are fatty adjuvants because of acyl chains. In general, these derivatives can have the following formula [29]: 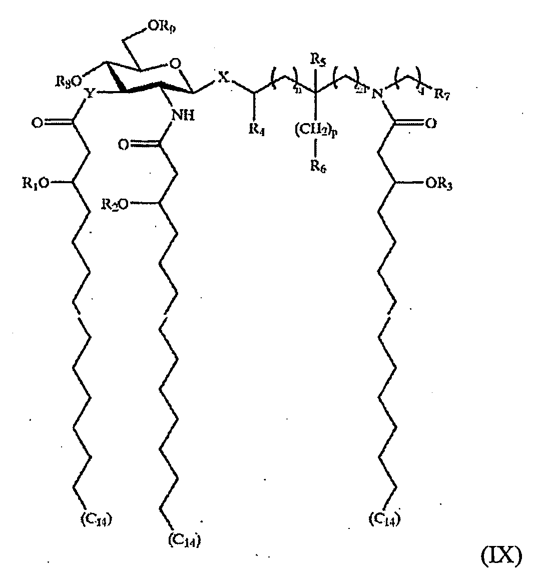 wherein:
- X represents an oxygen or sulfur atom in either the axial or equatorial position;
- Y represents an oxygen atom or NH group;
- "n", "m", "p" and "q" are the same or different and are integers from 0 to 6;
- R₁, R₂, and R₃, which may be the same or different, are fatty acyl residues having from 1 to about 20 carbon atoms and where one of R₁, R₂ or R₃ is optionally hydrogen;
- R₄ and R₅ are the same or different and are hydrogen or methyl;
- R₆ and R₇ are the same or different and are hydrogen, hydroxy, alkoxy, phosphono, phosphonooxy, sulfo, sulfooxy, amino, mercapto, cyano, nitro, formyl or carboxy and esters and amides thereof;
- R₈ and R₉ are the same or different and are phosphono or hydrogen, with preferably at least one of R₈ and/or R₉ being phosphono.

The configuration of the 3' stereogenic centers to which the normal fatty acyl residues are attached is R or S, but preferably R. The stereochemistry of the carbon atoms to which R₄ or R₅ are attached can be R or S. All stereoisomers, both enantiomers and diastereomers, and mixtures thereof, are considered to fall within the scope of the subject invention. Suitable adjuvants include salts of these compounds.

A preferred AGP compound is 'RC-529', which can be prepared as described in ref. 30:

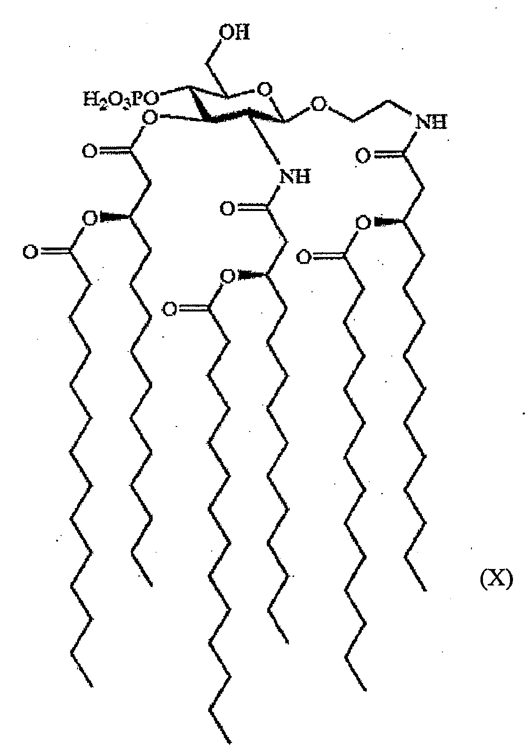

### Acyclic synthetic lipid A analogs

A lipid A analog that can be used as an adjuvant in the compositions of the invention is ER-112022 [31], a phospholipid dimer connected via an acyclic backbone:

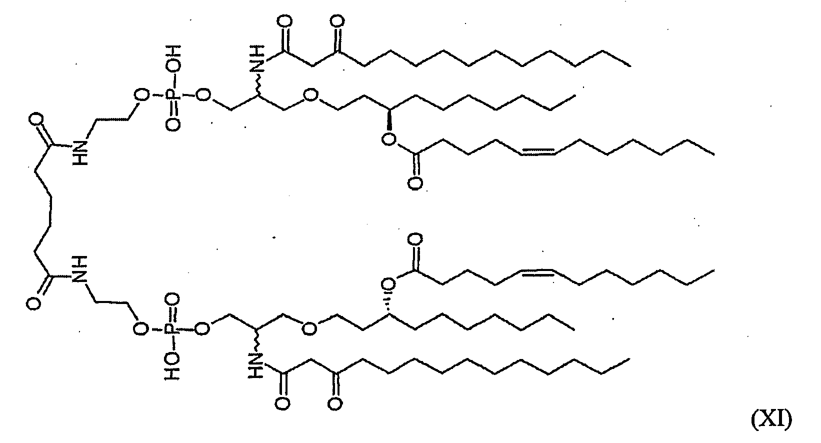

Each monomeric unit of ER-112022 contains 3 unique fatty groups that are bound indirectly to a phosphate diester. The fat groups include a 10-carbon ether chain, an unsaturated 12-carbon acyloxy chain bound to the ether chain, and a 14-carbon, b-oxo-amide chain linked closer to the phosphodiester. Thus, the compound has 3 unique features compared with naturally occurring lipid A from *E.coli:* (i) ER-112022 is devoid of a cyclic carbohydrate backbone; (ii) second, the phosphates are phosphodiesters incorporated within the confines of the structural backbone, unlike the phosphoesters on *E.coli* lipid A; and (iii) the structure is symmetrical (six symmetrically organized fatty acids on a noncarbohydrate backbone).

Similar useful compounds include those with formula XIV, XV or XVI, or salts thereof: 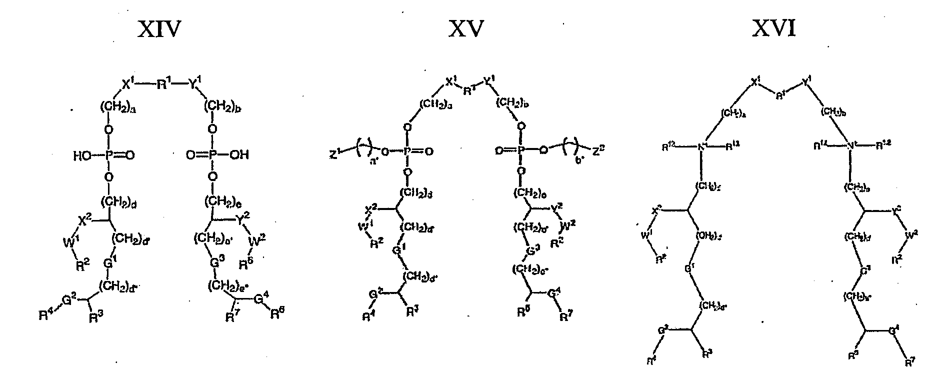 as defined in reference 32, such as 'ER 803058', 'ER 803732', 'ER 804053', ER 804058', 'ER 804059', 'ER 804442', 'ER 804680', 'ER 804764', ER 803022 or 'ER 804057' *e.g*.: 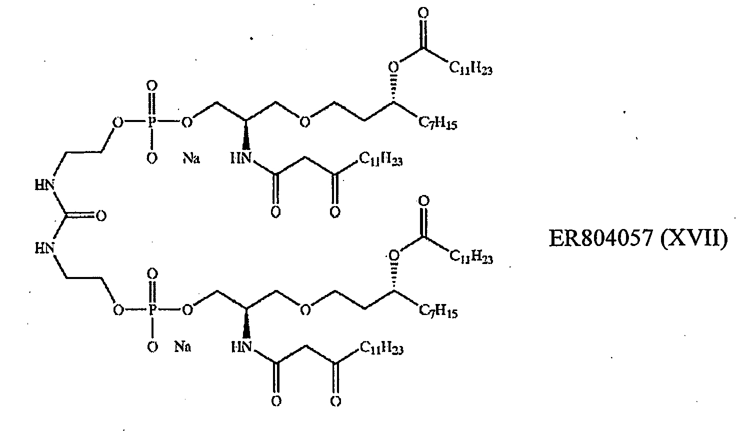 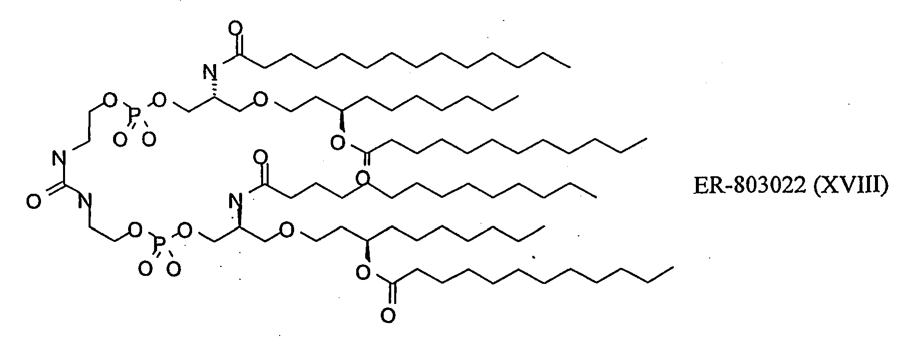 Compounds containing lipids linked to a phosphate-containing acyclic backbone can also be used as adjuvants, such as the TLR4 antagonist E5564 [33,34]:

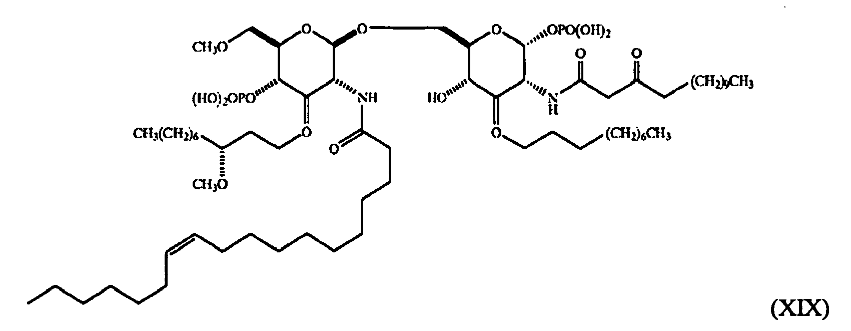

### The surfactant

The invention uses surfactant during antigen purification, but at controlled levels relative to the fatty adjuvant.

The surfactant will typically be a non-ionic surfactant, particularly those that are already found in vaccine formulations. Organic surfactants are preferred. These are typically the reaction product of an alkylene oxide (*e.g*. ethylene oxide) with a fatty alcohol, fatty acid, alkylphenol, alkylamine or other appropriate compound having at least one active hydrogen atom. For most surfactants the most common alcohols, amines and acids have a carbon chain length in the range C₈-C₁₈. The most common alkylphenols are nonylphenol and octylphenol. Surfactants containing poly(oxyethene) residues are particularly preferred.

For example, the invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); polyoxyethylene fatty ethers derived from lauryl, cetyl, stearyl and oleyl alcohols (known as Brij surfactants), such as triethyleneglycol monolauryl ether (Brij 30); and sorbitan esters (commonly referred to as the SPANs), such as sorbitan trioleate (Span 85) and sorbitan monolaurate.

Two specific surfactants of interest are Triton X-100 and polysorbate 20. Preferred embodiments of the invention use an antigen component that comprises one of these two non-ionic surfactants.

The invention is particularly suitable for use with polysorbate 20. This surfactant has an established safety profile for administration to humans, including within vaccines.

Surfactants can be classified by their 'HLB' (hydrophile/lipophile balance). Preferred surfactants of the invention have a HLB of at least 10, preferably at least 15, and more preferably at least 16.

To avoid administering large doses of the surfactant to a patient, it is preferred that the concentration of the surfactant in the composition should be no more than 50µg/ml *e.g*. ≤40µg/ml, ≤35µg/ml, ≤30µg/ml, ≤25µg/ml, ≤20µg/ml, ≤15µg/ml, ≤10µg/ml, ≤5µg/ml, *etc.* A concentration of ≤20µg/ml is preferred.

### The antigen

The invention involves the use of a viral surface antigen. The invention is particularly suitable for use with antigens that are typically purified by using surfactants. These antigens are typically lipophilic. They will usually include at least one transmembrane region, which functions *in vivo* to localise the antigen within a lipid bilayer *e.g*. on the surface of a pathogen.

The antigen component includes a surfactant. Rather than being a simple mixture of antigen and surfactant, it is preferred that the antigen and surfactant should be in the form of a complex. Antigen/surfactant complexes include surfactant-stabilised liposomes containing antigen, and niosomes, which are vesicles formed from synthetic nonionic amphiphilic compounds. A preferred antigen/surfactant complex is a particulate hepatitis B surface antigen, purified in the presence of surfactant. Reference 35 describes how recombinant HBsAg particles can retain Tween 20 that was used during their purification (up to 25µg Tween 20 per 100µg HBsAg).

The most preferred antigen for use with the invention is thus the surface antigen of hepatitis B virus (HBV), in the form of substantially-spherical particles (average diameter of about 20nm), including a lipid matrix comprising both phospholipids and a non-ionic surfactant, such as polysorbate 20. Non-ionic surfactant can be incorporated into the particle during purification.

Hepatitis B virus (HBV) is one of the known agents which causes viral hepatitis. The HBV virion consists of an inner core surrounded by an outer protein coat or capsid, and the viral core contains the viral DNA genome. The major component of the capsid is a protein known as HBV surface antigen or, more commonly, 'HBsAg', which is typically a 226-amino acid polypeptide with a molecular weight of -24 kDa. All existing hepatitis B vaccines contain HBsAg, and when this antigen is administered to a normal vaccinee it stimulates the production of anti-HBsAg antibodies which protect against HBV infection.

For vaccine manufacture, HBsAg can be made in two ways. The first method involves purifying the antigen in particulate form from the plasma of chronic hepatitis B carriers, as large quantities of HBsAg are synthesized in the liver and released into the blood stream during an HBV infection. The second way involves expressing the protein by recombinant DNA methods. HBsAg for use with the method of the invention is recombinantly expressed in yeast cells. Suitable yeasts include *Saccharomyces* (such as *S.cerevisiae*), *Pichia* (such as *P.pastoris*) or *Hanensula* (such as *H.polymorpha*) hosts. As an alternative, the antigen can be expressed in recombinant mammalian *(e.g.* in Chinese hamster ovary (CHO). COS cells, Bu3 cells, *etc*.), insect *(e.g.* using baculovirus vectors), or plant cells. In general, however, yeast expression is used.

The yeast-expressed HBsAg is preferably non-glycosylated. Unlike native HBsAg (*i*.*e*. as in the plasma-purified product), yeast-expressed HBsAg is generally non-glycosylated, and this is the most preferred form of HBsAg for use with the invention, because it is highly immunogenic and can be prepared without the risk of blood product contamination. Yeast-expressed HBsAg particles may include phosphatidylinositol, which is not found in natural HBV virions. The particles may also include a non-toxic amount of LPS in order to stimulate the immune system [36].

Manly methods for purifying HBsAg are known in the art (*e.g*. see refs 37-62). Of these various process, the invention uses a non-ionic surfactant during purification, such that surfactant becomes incorporated into the final particulate HBsAg product. Using polysorbate 20 during disruption of recombinant yeast cells at the start of purification is a preferred way to introduce the surfactant into the HBsAg particles.

A preferred method for HBsAg purification involves, after cell disruption: ultrafiltration; size exclusion chromatography; anion exchange chromatography; ultracentrifugation; desalting; and sterile filtration. Lysates may be precipitated after cell disruption (*e.g*. using a polyethylene glycol), leaving HBsAg in solution, ready for ultrafiltration. Before or after the sterile filtration, it is possible to stabilise HBsAg particles by treatment with formaldehyde. Excess formaldehyde can then be removed by ultrafiltration or by chromatography. Further sterile filtration may be used.

The HBsAg is preferably from HBV subtype adw2.

In addition to the 'S' sequence, a surface antigen may include all or part of a pre-S sequence, such as all or part of a pre-S 1 and/or pre-S2 sequence.

Other viral surface antigens that can be used with the invention will generally be envelope glycoproteins from enveloped viruses. Viral surface antigens for use with the invention can include, but are not limited to:
- A retroviridae protein. Retroviridae include lentiviruses and spumaviruses. Viruses of interest include HTLV-I, HTLV-II, feline immunodeficiency virus (FIV), human immunodeficiency virus (HIV, including HIV-1 and HIV-2), simian immunodeficiency virus (SIV), chimpanzee foamy virus and human spumavirus.
- A paramyxoviridae protein, such as the F protein. Paramyxoviridae include (a) the Paramyxovirinae, which includes Paramyxoviruses, Rubulaviruses and Morbilliviruses and (b) the Pneumovirinae, which includes the Pneumoviruses. Viruses of interest include parainfluenza virus (PIV), human paramyxovirus, Rinderpest virus, Peste Des Petit Ruminant virus, Measles virus, Mumps virus, respiratory syncytial virus (RSV), Nipah Virus, Hendra Virus, Equine Morbillivirus (EMV), Lyssavirus and Menangle virus.
- A filoviridae protein. Filoviridae include the Marburg and Ebola viruses.
- A spike glycoproteins of a coronaviridae. Coronaviridae include coronaviruses and toroviruses. Viruses of interest include the human coronaviruses (including the SARS coronavirus), Avian infectious bronchitis virus, Feline infectious peritonitis virus, Murine hepatitis virus, Porcine epidemic diarrhea virus, Porcine hemagglutinating encephalomyelitis virus, Porcine transmissible gastroenteritis virus, and Berne virus.
- A rhabdoviridae protein, such as the G protein. Rhabdoviridae include Rhabdoviruses, Vesiculoviruses, Lyssaviruses, Ephemeroviruses, Cytorhabdoviruses and Nucleorhabdoviruses. Viruses of interest include vesicular stomatitis virus, rabies virus, mokola virus, bovine ephemeral fever virus.
- A togaviridae protein. Togaviridae include Alphaviruses and Rubiviruses. Viruses of interest include Sindbis virus, Eastern and Western encephalitis viruses, Semliki Forest virus, rubella virus, Aura virus, Babanki virus, Barmah Forest virus avis-A, bebaru virus, Buggy Creek virus, chikungunya virus, Everglades virus, Fort Morgan virus, getah virus, Highlands J virus, Kyzylagach virus, Mayaro virus, Middelburg virus, Mucambo virus, Ndumu virus, Ockelbo virus, o'nyong-nyong virus, Pixuna virus, Ross River virus, Sagiyama virus, Una virus, Venezuelan equine encephalitis virus, and Whataroa virus.
- An envelope ('E') glycoprotein of a flaviviridae. Flaviviridae include Flaviviruses, Pestiviruses and Hepaciviruses. Viruses of interest include dengue virus, hepatitis C virus, yellow fever virus, japanese encephalitis virus, west nile virus, St. Louis encephalitis virus, bovine diarrhea virus and tick-borne encephalitis (TBE) virus.
- A bunyaviridae protein. Bunyaviridae include Bunyaviruses, Nairoviruses, Phleboviruses, Hantaviruses, and Tospoviruses. Viruses of interest include bunyavirus, Bunyamwera virus, california encephalitis virus, La Cross virus, Hantaan virus, Sin Nombre virus, Crimean-congo hemorrhagic fever virus, Sandfly fever Sicilian virus and Rift valley fever virus.
- An arenaviridae protein. Arenaviridae include lymphocytic choriomeningitis virus, ippy virus and lassa virus.
- A hepadnaviridae protein (including HBsAg). Hepadnaviridae include orthohepadnaviruses and avihepadnaviruses. As well as human hepatitis B virus, this viral family includes ground squirrel hepatitis B virus, woodchuck hepatitis B virus, woolly monkey hepatitis B virus, arctic squirrel hepatitis virus, duck hepatitis B virus, heron hepatitis B virus, and Ross' goose hepatitis B virus.
- A herpesviridae protein. Herpesviridae include Simplexviruses, Varicelloviruses, Roseoloviruses, Cytomegaloviruses, Muromegaloviruses, Lymphocryptoviruess and Rhadinoviruses. Viruses of interest include the human herpes viruses, including Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HBV7), and Human Herpesvirus 8 (HHV8), *etc.* Suitable antigens may be selected from glycoproteins gB, gC, gD and gH (*e.g*. in HSV). HSV gD2 is particularly preferred.
- An Orthomyxoviridae protein. Orthomyxoviridae include influenza virus and thogoto viruses. Antigens from influenza viruses are preferred (influenza A, B or C virus), including surface antigens hemagglutinin (HA) and/or neuraminidase (NA).

These viral surface antigens are typically purified by using surfactants, and so the antigen component can include a surfactant, particularly if present in a particulate form including lipids.

The invention is also useful with particulate antigens based on hybrid or fusion proteins that comprise a viral surface antigen and a heterologous antigen. For instance, it is known to fuse the HBsAg sequence to heterologous antigens to exploit HBsAg's ability to assemble into particles.

For example, reference 63 reports fusions of HIV-1 gp120 to HBsAg to give a protein that spontaneously assembled into particles that resemble native HBsAg particles in size and density, consistent with a lipid composition of about 25% and a gp120 content of about 100 per particle. The gp120 was able to folds into its native conformation in the fusion, and retained its biological activity. Similarly, HIV gp41 epitopes have been improved by making internal fusions with HBsAg, and the fusion protein self-assembled in yeast into 22nm lipoprotein particles [64].

This approach has also been used for malaria vaccines. Reference 65 reports that epitopes of up to 61aa from the malaria gp190 antigen were inserted into the HBsAg sequence, and that the expressed hybrid particles could elicit an anti-gp190 immune response in animals. Reference 66 report an protein having 16 repeats of a 4-mer sequence of the circumsporozoite protein expressed as a fusion protein with HBsAg. Reference 67 reports the production in yeast of virus-like particles composed of Pfsl6 fused to HBsAg. Reference 68 discloses a hybrid antigen in which the circumsporozoite protein is fused to HBsAg. Reference 69 discloses a fusion of the C-terminal region of the merozoite surface 1 protein of *P vivax,* which formed immunogenic particles of 20-45 nm size. The use of HBsAg for presenting malarial antigens in self-assembling particulate form is therefore well known in the art.

Thus the invention can be used with hybrid antigens that comprise a viral surface antigen and a heterologous antigen. The heterologous antigen may be inserted into the viral surface antigen sequence, or may be fused to the N-terminus or C-terminus of the viral surface antigen sequence. If the native viral surface antigen can assemble into particles (*e.g*. HBsAg) then the insertion or fusion will not prevent this assembly.

In these hybrid proteins, the heterologous antigen may be from a bacterium, from a fungus, from a parasite, from a virus (but, by definition, the heterologous antigen is not HBsAg), *etc.* It is possible to include a complete heterologous antigen in the hybrid protein, but it is more usual to include an antigenic fragment of the antigen.

Particularly where the viral surface antigen is HBsAg, the heterologous antigen may be from HIV, *Plasmodium falciparum, Plasmodium vivax, Plasmodium malariae* or *Plasmodium ovale.* Suitable HIV antigens for making HBsAg hybrids include envelope glycoprotein gp120 or antigenic fragments thereof [63]. Suitable *P falciparum* antigens for making HBsAg hybrids may be based on a subunit of the circumsporozoite surface antigen ("CSP") *e.g*. they may include between 3 and 20 repeats of its NANP motif (SEQ ID NO: 2), and/or they may include the C-terminal region of CSP (but typically not including the final 12 amino acids from the C-terminal). For example, the invention may use the antigen known as "RTS", which contains a large portion of the C-terminal of CSP from the NF54 or 7G8 isolate of *P.falcipanum* (amino acids 210 to 398, which includes 19 NANP repeats and the T cell epitope region at amino acids 367 to 390), fused to the N-terminus of HBsAg by four amino acids of the preS2 portion of HBsAg. When expressed in yeast, RTS forms particles that include lipids (primarily phospholipid) in addition to protein. The sequence of RTS can thus contain: (i) a N-terminus methionine residue; (ii) Met-Ala-Pro; (iii) 189 amino acids corresponding either to amino acids 210-398 of CS protein from *P falciparum* 7G8 or to amino acids 207-395 of CS protein from *P.falciparum* NF54; (iv) Arg or Gly; (v) Pro-Val-Thr-Asn from hepatitis B Pre-S2 protein; and (vi) HBsAg. From the 7G8 isolate, full-length RTS has the sequence given as SEQ ID NO: 1 in reference 70 (see also Figure 5 of reference 71):

The hybrid protein can be expressed in yeast, using a sequence encoding SEQ ID NO:1. It is preferred to co-express the hybrid protein in yeast with normal HBsAg. This approach has previously been used with RTS, and the product of co-expression is referred to as "RTS,S". A RTS:S ratio of about 1:4 is useful. Expression in *S.cerevisiae* yeast is preferred, using a plasmid having a sequence encoding the hybrid protein, and including: (1) an upstream promoter from a glyceraldehyde-3-phosphate dehydrogenase gene, for controlling expression of the coding sequence; and (2) an ARG3 transcription terminator downstream of the coding sequence. The plasmids will also typically include: (3) a LEU2 selection marker; (4) a 2µ plasmid sequence; and (5) an origin of replication functional in *Escherichia coli.*

RTS,S may be combined with other malarial antigens, such as thrombospondin-related anonymous protein (TRAP). A preferred fatty adjuvant for use with RTS,S includes an oil-in-water emulsion, 3d-MPL and a QS-21 saponin.

### Fatty adjuvant and surfactant ratio

The weight ratio in the composition of the fatty adjuvant to the antigen's surfactant is less than 1000:1.

For a MF59 adjuvant, the weight ratio is based on the amount of squalene. For a composition containing 0.1 µg/ml surfactant in an antigen component, the squalene concentration will be less than 100µg/ml. For a composition containing 1µg/ml surfactant in an antigen component, the squalene concentration will be less than 1mg/ml. For a composition containing 10µg/ml surfactant in an antigen component, the squalene concentration will be less than 10mg/ml, whereas the squalene concentration in a typical MF59-adjuvanted composition would be 43mg/ml (*i.e*. 4300:1).

For a 3D-MPL adjuvant, the weight ratio is based on the total amount of 3D-MPL *i.e*. including all of the different acyl forms that may be present. For a composition containing 0.1 µg/ml surfactant in an antigen component, the total 3D-MPL concentration will be less than 100µg/ml. For a composition containing 1µg/ml surfactant in an antigen component, the total 3D-MPL concentration will be less than 1mg/ml.

The 1000:1 ratio is a maximum. To further reduce the potential for interference between oil in an adjuvant and surfactant in an antigen, the ratio can be reduced. Thus the ratio may be ≤500:1, ≤400:1, ≤300:1, ≤200:1, ≤100:1, ≤50:1, or even ≤25:1. At a ≤100:1 ratio, a composition containing 10µg/ml surfactant in an antigen component would have an oil content (*e.g*. squalene or 3D-MPL content) of ≤lmg/ml. At a ≤25:1 ratio, a composition containing 10µg/ml surfactant in an antigen component would have an oil content (*e.g*. squalene or 3D-MPL content) of <250µg/ml; conversely, a composition comprising 100µg/ml 3D-MPL would have no less than 4µg/ml surfactant as part of the antigen.

The ratio is preferably greater than 1.5:1 *e.g*. ≥2:1, ≥2.5:1, ≥3:1, ≥4:1, ≥5:1 or more.

For 3D-MPL, a ratio between 2.5:1 and 25:1 is preferred, more preferably between 2.5:1 and 10:1, and even more preferably between 2.5:1 and 5:1. Thus a composition containing 10µg/ml surfactant in an antigen component would have a 3D-MPL content of between 25µg/ml and 250µg/ml, preferably between 25µg/ml and 100µg/ml, and even more preferably between 25µg/ml and 50µg/ml; conversely, a composition comprising 100µg/ml 3D-MPL would include an antigen having a surfactant content of between 4µg/ml and 40µg/ml, preferably between 10µg/ml and 40µg/ml, and even more preferably between 20µ/ml and 40µg/ml.

### The immunogenic composition

As well as containing antigen (and a fatty adjuvant (and a low level of surfactant), compositions of the invention may comprise carriers, adjuvants, excipients, buffers, *etc.,* as described in more detail below. These non-antigenic components may have various sources. For example, they may be present in one of the antigen or adjuvant materials that is used during manufacture or may be added separately from those components.

Preferred compositions of the invention include one or more pharmaceutical carrier(s) and/or excipient(s).

To control tonicity, it is preferred to include a physiological salt, such as a sodium salt. Sodium chloride (NaCl) is preferred, which may be present at between 1 and 20 mg/ml.

Compositions will generally have an osmolality of between 200 mOsm/kg and 400 mOsm/kg, preferably between 240-360 mOsm/kg, and will more preferably fall within the range of 280-320 mOsm/kg. Osmolality has previously been reported not to have an impact on pain caused by vaccination [72], but keeping osmolality in this range is nevertheless preferred.

Compositions of the invention may include one or more buffers. Typical buffers include: a phosphate buffer; a Tris buffer; a borate buffer; a succinate buffer; a histidine buffer; or a citrate buffer. Buffers will typically be included in the 5-50mM range, and preferably in the 5-20mM range.

The pH of a composition of the invention will generally be between 5.0 and 7.5, and more typically between 5.0 and 6.0 for optimum stability, or between 6.0 and 7.0. The process of the invention may therefore include a step of adjusting the pH of the bulk vaccine prior to packaging into dosage containers. A vaccine containing diphtheria and tetanus toxoids preferably has a pH ≥6, to avoid the risk of toxicity reversion in the toxoids (particularly in the diphtheria toxoid), and thus a vaccine containing toxoids and HBsAg antigens preferably has a pH between 6.0 and 7.0. For other vaccines, including monovalent HBsAg vaccines, a pH <6 may be acceptable.

Compositions of the invention are preferably sterile.

Compositions of the invention are preferably non-pyrogenic *e.g*. containing <1 EU (endotoxin unit, a standard measure) per dose, and preferably <0.1 EU per dose.

Compositions of the invention are preferably gluten free.

If adsorbed HBsAg is used, the final vaccine product may be a suspension with a cloudy appearance. This appearance means that microbial contamination is not readily visible, and so the vaccine preferably contains an antimicrobial agent. This is particularly important when the vaccine is packaged in multidose containers. Preferred preservatives for inclusion are 2-phenoxyethanol and/or thimerosal. It is recommended, however, not to use mercurial preservatives during the process of the invention, even though thimerosal is found in many existing HBV vaccines. For safety, however, it is preferred that the final composition contains less than about 25 ng/ml mercury. More preferably, the final vaccine product contains no detectable thimerosal. This will generally be achieved by removing the mercurial preservative from an antigen preparation prior to its addition in the process of the invention or by avoiding the use of thimerosal during the preparation of the components used to make the composition. HBsAg may be subjected to dialysis (*e.g*. with cysteine) to remove any mercurial preservatives such as thimerosal that may have been used during HBsAg preparation [73,74].

During manufacture, dilution of components to give desired final concentrations will usually be performed with WFI (water for injection).

The concentration of any aluminium phosphate in a composition of the invention, expressed in terms of Al³⁺, is preferably less than 5 mg/ml *e.g.* ≤4 mg/ml, ≤3 mg/ml, ≤2 mg/ml, ≤1 mg/ml, *etc.*

The concentration of HBsAg in a composition of the invention is preferably less than 100 µg/ml *e.g.* ≤90 µg/ml, ≤80 µg/ml, ≤70 µg/ml, ≤65 µg/ml, ≤60 µg/ml, ≤55 µg/ml, ≤50 µg/ml, ≤45 µg/ml, ≤40 µg/ml, *etc.* A concentration of about 40 µg/ml or about 20 µg/ml is typical.

Compositions of the invention are preferably administered to patients in 0.5ml doses. References to 0.5ml doses will be understood to include normal variance *e.g.* 0.5ml±0.05ml.

The invention can provide bulk material which is suitable for packaging into individual doses, which can then be distributed for administration to patients. Concentrations mentioned above are typically concentrations in final packaged dose, and so concentrations in bulk vaccine may be higher (*e.g.* to be reduced to final concentrations by dilution).

Compositions of the invention will generally be in aqueous form.

### Packaging compositions of the invention

After combining the antigen and adjuvant, the processes of the invention may comprise a step of extracting and packaging a 0.5ml sample of the mixture into a container. For multidose situations, multiple dose amounts will be extracted and packaged together in a single container. As mentioned above, in an alternative arrangement the antigen and adjuvant are packaged separately, for extemporaneous mixing at the point of use.

The processes of the invention may comprise the further step of packaging the vaccine into containers for use. Suitable containers include vials and disposable syringes (preferably sterile ones).

where a composition of the invention is packaged into vials, these are preferably made of a glass or plastic material. The vial is preferably sterilized before the composition is added to it. To avoid problems with latex-sensitive patients, vials are preferably sealed with a latex-free stopper. The vial may include a single dose of vaccine, or it may include more than one dose (a 'multidose' vial) *e.g.* 10 doses. When using a multidose vial, each dose should be withdrawn with a sterile needle and syringe under strict aseptic conditions, taking care to avoid contaminating the vial contents. Preferred vials are made of colorless glass.

A vial can have a cap (*e.g.* a Luer lock) adapted such that a pre-filled syringe can be inserted into the cap, the contents of the syringe can be expelled into the vial, and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed.

Where the composition is packaged into a syringe, which is preferred, the syringe will not normally have a needle attached to it, although a separate needle may be supplied with the syringe for assembly and use. Safety needles are preferred. 1-inch 23-gauge, 1-inch 25-gauge and 5/8-inch 25-gauge needles are typical. Syringes may be provided with peel-off labels on which the lot number and expiration date of the contents may be printed, to facilitate record keeping. The plunger in the syringe preferably has a stopper to prevent the plunger from being accidentally removed during aspiration. A rubber butyl plunger stopper is preferred. The syringes may have a latex rubber cap and/or plunger. Disposable syringes contain a single dose of vaccine. The syringe will generally have a tip cap to seal the tip prior to attachment of a needle, and the tip cap is preferably made of butyl rubber. If the syringe and needle are packaged separately then the needle is preferably fitted with a butyl rubber shield. Grey butyl rubber is preferred. Preferred syringes are those marketed under the trade name "Tip-Lok"™.

Where a glass container (*e.g.* a syringe or a vial) is used, then it is preferred to use a container made from a borosilicate glass rather than from a soda lime glass.

After a composition is packaged into a container, the container can then be enclosed within a box for distribution *e.g.* inside a cardboard box, and the box will be labeled with details of the vaccine *e.g.* its trade name, a list of the antigens in the vaccine (*e.g.* 'hepatitis B recombinant', *etc.*), the presentation container (*e.g.* 'Disposable Prefilled Tip-Lok Syringes' or '10 x 0.5 ml Single-Dose Vials'), its dose (*e.g.* 'each containing one 0.5ml dose'), warnings (*e.g.* 'For Adult Use Only' or 'For Pediatric Use Only'), an expiration date, an indication (*e.g.* 'active immunisation against hepatitis B virus (HBV) infection caused by all known subtypes for patients with renal insufficiency (including pre-haemodialysis and haemodialysis) patients, from the age of 15 years onwards', *etc.*), a patent number, *etc.* Each box might contain more than one packaged vaccine *e.g.* five or ten packaged vaccines (particularly for vials). If the vaccine is contained in a syringe then the box may show a picture of the syringe.

The vaccine may be distributed together (*e.g.* in the same box) with a leaflet including details of the vaccine *e.g.* instructions for administration, details of the antigens within the vaccine, *etc.* The instructions may also contain warnings *e.g.* to keep a solution of adrenaline readily available in case of anaphylactic reaction following vaccination, *etc.*

The packaged vaccine is preferably stored at between 2°C and 8°C. It should not be frozen.

### Methods of treatment and Administration of the vaccine

Compositions of the invention are suitable for administration to human patients, and the invention provides a method of raising an immune response in a patient, comprising the step of administering a composition of the invention to the patient.

The invention also provides the use of (i) a viral surface antigen component that includes a surfactant and (ii) a fatty adjuvant, in the manufacture of a medicament for administering to a patient, wherein the weight ratio of the fatty adjuvant in (ii) to surfactant in (i) is less than 1000:1.

Immunogenic compositions of the invention are preferably vaccines *e.g.* for use in the prevention and/or treatment of hepatitis B virus infection. Patients who have received compositions of the invention preferably have a serum anti-HBsAg GM titer of ≥500mIU/ml, measured 6 weeks after the first immunisation. More preferably, the titer is ≥500mIU/ml, when measured after 12 months.

The compositions are particularly useful for protecting against and/or treating hepatitis B virus infections in patients for whom existing adjuvanted vaccines (such as the ENGERIX B™ product) are ineffective. Sub-groups of patients for whom the compositions are particularly suitable include: immunocompromised patients; hemodialysis patients; pre-hemodialysis patients; patients with renal insufficiency; patients with renal failure; patients with early renal failure before they require hemodialysis; patients awaiting liver transplantation *e.g.* on waiting lists; patients with end-stage renal failure; patients who have received an organ transplant (particularly a liver transplant) *e.g.* in the 6 month period preceding a first dose of the vaccine of the invention; patients who are receiving (or have been receiving *e.g.* in the 6 month period preceding a first dose of the vaccine of the invention) hepatitis B immunoglobulin (HBIg) treatment; patients with a HLA DQ2 haplotype [75]; patients with a HLA DR3 haplotype [75]; patients with a HLA DR7 haplotype [75]; patients with the with the HLA allele DQB 1*0202 [76]; patients infected with HIV; chronic HBV carriers; patients who have recently received a blood transfusion; patients receiving immunosuppressive drugs; patients suffering from AIDS; patients with ascites; patients with cirrhosis; patients with encephalopathy; patients receiving interferon therapy, and in particular ifn-α; patients who smoke cigarettes; patients who smoke cigars; patients with a body mass index ≥30 kg/m²; and patients who have received a HBsAg vaccine but who have not seroconverted (*e.g.* they have a serum anti-HBsAg titer of <10 mIU/ml after a standard primary dosing schedule, such as 3 doses of ENGERIX B™).

These patients may have a creatinine clearance rate of less than 30ml/min (the normal healthy range being ∼100-140 ml/min in males and 90-130 ml/min in females). Patients are preferably at least 15 years old *e.g.* between 15-40 years, between 15-60 years, between 40-60 years, or even over 60. Patients aged over 55 can usefully be treated regardless of any underlying illness.

Compositions of the invention can be administered by intramuscular injection *e.g.* into the deltoid.

Where compositions of the invention include an aluminium-based adjuvant, settling of components may occur during storage. The composition should therefore be shaken prior to administration to a patient. The shaken composition will be a turbid white suspension.

### Preferred processes and vaccines of the invention

A preferred process for preparing an immunogenic composition comprises the steps of combining (i) an HBsAg component that includes polysorbate 20 and (ii) an adjuvant component comprising 3D-MPL adsorbed to an aluminium phosphate, to give a composition in which the weight ratio of 3D-MPL to polysorbate 20 is less than 1000:1.

A preferred immunogenic composition is one wherein: (a) the composition comprises HBsAg, polysorbate 20, 3D-MPL and an aluminium phosphate adjuvant wherein the polysorbate 20 is incorporated into HBsAg particles during purification; and (b) the weight ratio of the 3D-MPL to polysorbate 20 is less than 1000:1. The 3D-MPL and the HBsAg are preferably adsorbed to the aluminium phosphate. The HBsAg concentration is about 40µg/ml. The 3D-MPL concentration is about 100µg/ml. The Al³⁺ concentration is about 1mg/ml.

Another preferred composition comprises (i) HBsAg, purified from *S.cerevisiae*, and (ii) an adjuvant comprising a mixture of aluminium phosphate and 3D-MPL. The HBsAg concentration is about 40µg/ml. The 3D-MPL concentration is about 100µg/ml. The Al³⁺ concentration is about 1mg/ml. HBsAg includes polysorbate 20, and the oil:surfactant weight ratio (*i.e.* the 3D-MPL:polysorbate 20 weight ratio) is between 2.5:1 and 100:1 *i.e.* the polysorbate 20 is present at a level between 1µg/ml and 40µg/ml (*i.e.* between 2.5µg and 100µg polysorbate 20 per 100µg HBsAg). The ratio is preferably is between 2.5:1 and 25:1 *i.e.* the polysorbate 20 is present at a level between 4µg/ml and 40µg/ml. The 3D-MPL and the HBsAg are both adsorbed to the aluminium phosphate.

### General

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.* As described above, components can be mixed during manufacture, or extemporaneously at the time of use.

where an antigen is described as being "adsorbed" to an adjuvant, it is preferred that at least 50% (by weight) of that antigen is adsorbed *e.g.* 50%, 60%, 70%, 80%, 90%, 95%, 98% or more. It is preferred that HBsAg is totally adsorbed *i.e.* none is detectable in supernatant.

Where animal (and particularly bovine) materials are used in the culture of cells, they should be obtained from sources that are free from transmissible spongiform encaphalopathies (TSEs), and in particular free from bovine spongiform encephalopathy (BSE).

Butyl rubbers include chlorobutyl and bromobutyl rubbers.

It will be appreciated that ionisable groups may exist in the neutral form shown in formulae herein, or may exist in charged form *e.g.* depending on pH. Thus a phosphate group may be shown as -P-O-(OH)₂, this formula is merely representative of the neutral phosphate group, and other charged forms are encompassed by the invention. Similarly, sugar rings can exist in open and closed form and, while closed forms are shown in structural formulae herein, open forms are also encompassed by the invention.

### MODES FOR CARRYING OUT THE INVENTION

Three different HBsAg preparations were studied:
- HBsAg expressed in *S.cerevisiae* cells, purified using non-ionic surfactant.
- HBsAg expressed in *Hanensula* yeast cells, purified using non-ionic surfactant.
- HBsAg expressed in CHO cells, with preS2 sequence, purified without use of surfactants.

Two different adjuvants were studied:
- Aluminium hydroxide (1 mg/ml)
- MF59 in citrate buffer (13 mM)

Six formulations were prepared, each with 20µg/ml HBsAg, 0.15M NaCl and 0.01% merthiolate:

| | **A** | **B** | **C** | **D** | **E** | **F** |
|---|---|---|---|---|---|---|
| **HBsAg** | CHO | CHO | *Hanensula* | *Hanensula* | *S.cerevisiae* | *S.cerevisiae* |
| **Adjuvant** | Al Hydrox | MF59 | Al Hydrox | MF59 | Al Hydrox | MF59 |
| **NaCl** | 0.15M | 0.15M | 0.15M | 0.15M | 0.15M | 0.15M |
| **Merthiolate** | 0.01% | 0.01% | 0.01% | 0.01% | 0.01% | 0.01% |
| **Surfactant** | - | - | + | + | + | + |

MF59 has been reported to enhance the antibody response in primates to recombinant HBsAg [77]. Formulations A to D were used to immunise african green monkeys. Groups of 6 monkeys were immunised intramuscularly on day 0 and day 28. Bleeding were taken at time 0 and then every 2 weeks till week 8, and anti-HBsAg titres (GMT) were measured by ELISA. Titres were normalised to 1.0, which was the day 14 value in each group, and the normalised results were as follows:

| | **A** | **B** | **C** | **D** |
|---|---|---|---|---|
| ***Host*** | CHO | CHO | Yeast | Yeast |
| ***Adjuvant*** | *Al Hydrox* | *MF59* | *Al Hydrox* | *MF59* |
| ***Surfactant*** | - | - | + | + |
| **Day 14** | 1.0 | 1.0 | 1.0 | 1.0 |
| **Day 28** | 0.9 | 0.8 | 1.3 | 0.4 |
| **Day 42** | 19.2 | 159.8 | 108.8 | 85.2 |
| **Day 56** | 10.8 | 32.6 | 61.3 | 24.5 |

Looking at the day 42 and day 56 titres, these data show that the MF59 adjuvant increased titres much more markedly than the aluminium adjuvant for CHO-expressed antigen (160-fold increase vs. 20-fold increase at day 42), but the situation was reversed for the *Hanensula*-expressed antigen (85-fold increase vs. 110-fold increase at day 42).

Similar experiments were performed in baboons, using formulations B, E and F. GMT values were measured 14 days after the first injection and, relative to the group F value, were as follows:

| | **A** | **B** | **E** | **F** |
|---|---|---|---|---|
| ***Surfactant*** | - | - | + | + |
| ***Adjuvant*** | *Al Hydrox* | *MF59* | *Al Hydrox* | *MF59* |
| **Day 14** | - | 13.5 | 4.5 | 1.0 |

Here, using MF59 instead of the alum adjuvant leads to a lower GMT response (compare groups E and F), whereas an increase would be expected (group B and reference 77).

Anti-HBsAg responses 28 days after first injection using formulations E and F were also compared in rhesus monkeys, african green monkeys and C3H mice (NB: mice received 60% of the HBsAg dose). As well as measuring GMT values, the number of responders was also measured *i.e.* animals showing a titre of >10mIU/ml. Results, normalised to group F, were as follows:

| | **GMT** | | **Responders** | |
|---|---|---|---|---|
| | **E** | **F** | **E** | **F** |
| ***Adjuvant*** | *Al Hydrox* | *MF59* | *Al Hydrox* | *MF59* |
| **Rhesus** | 4.2 | 1.0 | 60% | 20% |
| **AGM** | 110.0 | 1.0 | 100% | 50% |
| **Mice** | 0.1 | 1.0 | 94% | 100% |

In contrast to mice, therefore, using MF59 as the adjuvant in primates resulted in a substantial decrease both in GMT values and in responder levels for the *S.cerevisiae*-expressed HBsAg.

Finally, the increase in GMT resulting from booster vaccination with formulations E and F was measured in seropositive african green monkeys. Titres were measured before the booster dose and then again 28 days later, and the increase in titres was as follows:

| **Group** | **Adjuvant** | **Increase (x)** |
|---|---|---|
| **E** | Al Hydrox | 91.5 x |
| **F** | MF59 | 19.1 x |

As well as giving a lower fold-increase than the aluminium hydroxide adjuvant, MF59 gave a lower GMT value 28 days after the booster dose.

Overall, therefore, MF59 seems to be a worse adjuvant than aluminium hydroxide for yeast-expressed HBsAg in primates, but a better adjuvant for CHO-expressed HBsAg. These results may be explained by an interaction between (a) residual surfactant in HBsAg purified from yeast and (b) excess squalene oil in the MF59 adjuvant. As an oil, squalene is subject to the action of surfactants in aqueous conditions, and *vice versa*, and it is proposed that the surfactant in the antigen is incompatible with the oil in the adjuvant. This incompatibility can be dealt with either by purifying the HBsAg without using detergents (*e.g.* as in the CHO-expressed material), or by ensuring that the oil is not present at such an excess that it interferes with the surfactant in the antigen.

In a MF59-adjuvanted HBsAg composition, one volume of MF59 is combined with one volume of antigen solution. In a 100ml volume, there will thus be 50ml MF59 and 50ml HBsAg solution. This 100ml contains 2.15g squalene from the MF59 and, at a HBsAg concentration of 40µg/ml, it contains 2mg HBsAg. At a polysorbate 20 concentration of 25µg per 100µg HBsAg [35], the 100ml contains 0.5mg polysorbate 20. The oil:surfactant ratio in a typical MF59-adjuvanted vaccine is thus 4300:1. By reducing the oil excess to be less than 1000:1, the interaction between oil in the adjuvant and surfactant in the antigen can be substantially reduced, thereby reducing interference.

It will be understood that the invention has been described by way of example only and modifications may be made whilst remaining within the scope of the invention.

### REFERENCES

[1] Vaccine Adjuvants: Preparation Methods and Research Protocols (Volume 42 of Methods in Molecular Medicine series). ISBN: 1-59259-083-7. Ed. O'Hagan.
*[2]* Vaccine Design: The Subunit and Adjuvant Approach (eds. Powell & Newman) Plenum Press 1995 (ISBN 0-306-44867-X).
[3] WO90/14837.
[4] Podda & Del Giudice (2003) Expert Rev Vaccines 2:197-203.
[5] US patent 6,299,884.
[6] US patent 6,451,325.
[7] Allison & Byars (1992) Res Immunol 143:519-25.
[8] Hariharan et al. (1995) Cancer Res 55:3486-9.
[9] WO95/11700.
[10] US patent 6,080,725.
[11] WO2005/097181.
[12] UK patent application GB-A-2220211.
[13] Myers et al. (1990) pages 145-156 of Cellular and molecular aspects of endotoxin reaction.
[14] Ulrich (2000) Chapter 16 (pages 273-282) of reference 1.
[15] Johnson et al. (1999) J Med Chem 42:4640-9.
[16] Baldrick et al. (2002) Regulatory Toxicol Pharmacol 35:398-413.
[17] WO 94/21292.
[18] WO94/00153.
[19] WO97/01640/
[20] WO96/26741.
[21] WO93/19780.
[22] US patent 6,375,945.
[23] WO00/00462.
[24] Meraldi et al. (2003) Vaccine 21:2485-2491.
[25] Pajak et al. (2003) Vaccine 21:836-842.
[26] Brandenburg et al. (2000) Eur J Biochem 267 :3370-7.
[27] Johnson et al. (1999) Bioorg Med Chem Lett 9:2273-2278.
[28] Evans et al. (2003) Expert Rev Vaccines 2:219-229.
[29] US patent 6,764,840.
[30] US patent application 2005/0107600.
[31] Lien et al. (2001) J. Biol. Chem 276:1873-80.
[32] WO03/011223.
[33] Wong et al. (2003) J Clin Pharmacol 43(7):735-42.
[34] US2005/0215517.
[35] Costa et al. (1997) Rev. Inst. Med. trop. S. Paulo v.39 n.1 São Paulo Ene.
[36] Vanlandschoot et al. (2005) J Gen Virol 86:323-31.
[37] Hilleman (1993) pages 17-40 of Hepatitis B vaccines in clinical practice ISBN 0-8247-8780-3.
[38] Gerin et al. (1969) J Virol 4:763.
[39] Gerin et al. (1969) J Virol 7:569.
[40] Siebke et al. (1972) Acta Pathol Microbiol Scan sect B 80:935.
[41] Pillot et al. (1976) J Clin Microbiol 4:205.
[42] Duimel & Krijnen (1972) Vox Sang 23:249.
[43] Neurath et al. (1974) PNAS USA 71:2663.
[44] Charm & Wong (1975) Biotechnol Bioeng 16:593.
[45] US patent 4,857,317.
[46] US patent 4,683,294.
[47] Houwen et al. (1975) J Immunol Methods 8:185.
[48] Sitrin et al. (1993) pages 83-101 of Hepatitis B vaccines in clinical practice ISBN 0-8247-8780-3.
[49] Wampler et al. (1985) PNAS USA 82:6830-4.
[50] Chi et al. (1994) Ann N Y Acad Sci 721:365-73.
[51] Agraz et al. (1994) J Chromatogr A 672:25-33.
[52] Mason et al. (1992) PNAS USA 89:11745-9.
[53] Deml et al. (1999) J Virol Methods 79:205-17.
[54] Ibarra et al. (1999) J Chromatogr B Biomed Sci Appl 735:271-7.
[55] WO90/10058.
[56] Japanese patent application JP63239234.
[57] US patent 4,694,074.
[58] EP-0341733.
[59] US patent 5,242,812.
[60] Russian patent application RU-2128707.
[61] Hardy et al. (2000) J Biotechnol 77:157-67.
[62] Dogan et al. (2000) Biotechnol Prog 16:435-41.
[63] Berkower et al. (2004) Virology 321(1):75-86.
[64] Eckhart et al. (1996) J Gen Virol 77:2001-8.
[65] von Brunn et al. (1991) Vaccine 9(7):477-84.
[66] Vreden et al. (1991) Am J Trop Med Hyg 45(5):533-8.
[67] Moelans et al. (1995) Mol Biochem Parasitol 72(1-2):179-92.
[68] Stoute et al. (1997) N Engl J Med 336(2):86-91.
[69] Wunderlich & del Portillo (2000) Mol Med 6(3):238-45.
[70] US patent 5,928,902
[71] WO93/10152.
[72] Nony et al. (2001) Vaccine 27:3645-51.
[73] WO02/12287
[74] WO03/066094.
[75] Desombere et al. (1998) Tissue Antigens 51:593-604.
[76] McDermott et al. (1997) Tissue Antigens. 50:8-14.
[77] Traquina et al. (1996) J Infect Dis 174:1168-75.

## Claims

1. A process for preparing an immunogenic composition, comprising the steps of combining (i) a viral surface antigen component that includes a surfactant and (ii) a fatty adjuvant component, to give a composition in which the weight ratio of the fatty adjuvant to the surfactant is less than 1000:1.

2. The process of claim 1, wherein the fatty adjuvant comprises a metabolisable oil.

3. The process of claim 2, wherein the fatty adjuvant comprises a sub-micron oil-in-water emulsion of squalene and polysorbate 80.

4. The process of claim 1, wherein the fatty adjuvant comprises a molecule comprising a fatty acid moiety.

5. The process of claim 4, wherein the fatty adjuvant is a 3-deoxyacylated monophosphoryl lipid A (`3D-MPL').

6. The process of claim 5, wherein the 3D-MPL is a mixture of molecules varying by their acylation.

7. The process of claim 5 or claim 6, wherein the fatty adjuvant includes:

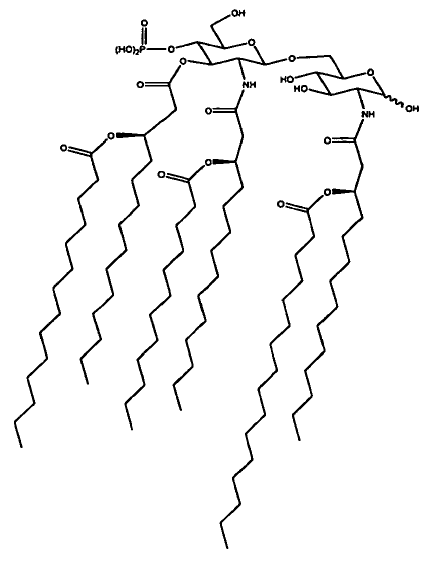

8. The process of any one of claims 5 to 7, wherein the 3D-MPL is in the form of particles.

9. The process of claim 8, wherein the particles can be filter sterilised.

10. The process of any one of claims 5 to 9, wherein the 3D-MPL is in combination with an aluminium salt.

11. The process of claim 10, wherein the aluminium salt is an aluminium phosphate.

12. The process of claim 11, wherein the 3D-MPL is adsorbed onto the aluminium salt.

13. The process of any preceding claim, wherein the surfactant is a polyoxyethylene sorbitan ester.

14. The process of claim 13, wherein the ester is polysorbate 20.

15. The process of claim 13 or claim 14, wherein the concentration of surfactant no more than 50µg/ml.

16. The process of any preceding claim, wherein the viral surface antigen is a particulate hepatitis B surface antigen ('HBsAg'), purified in the presence of surfactant.

17. The process of claim 16, wherein the HBsAg is expressed in a yeast cell.

18. The process of claim 17, wherein the yeast is a *Saccharomyces cerevisiae*.

19. The process of any one of claims 16 to 18, wherein the HBsAg is non-glycosylated and/or includes phosphatidylinositol.

20. The process of any one of claims 16 to 19, wherein the HBsAg is from subtype adw2 of hepatitis B virus.

21. The process of any preceding claim, wherein the antigen is a hybrid protein comprising a viral surface antigen and a heterologous antigen.

22. The process of claim 21, wherein the viral surface antigen is HBsAg and the heterologous antigen is a malaria antigen.

23. The process of claim 22, wherein the hybrid protein comprises HBsAg and a fragment of the circumsporozoite protein of *Plasmodium falciparum*.

24. The process of claim 23, wherein the hybrid protein comprises a C-terminal portion of a *P.falciparum* circumsporozoite protein of Plasmodium falciparum, four or more tandem repeats of the circumsporozoite protein immunodominant region, and HBsAg.

25. The process of any preceding claim, wherein the weight ratio of the fatty adjuvant to the surfactant is less than 500:1.

26. The process of any preceding claim, wherein the weight ratio of the fatty adjuvant to the surfactant is less than 50:1.

27. The process of any preceding claim, wherein the fatty adjuvant is a 3D-MPL and the weight ratio of the fatty adjuvant to the surfactant is between 2.5:1 and 25:1.

28. The process of any preceding claim, wherein the composition has an osmolality of between 200 mOsm/kg and 400 mOsm/kg.

29. The process of any preceding claim, wherein the composition includes a phosphate buffer.

30. The process of any preceding claim, wherein the composition has a pH between 6.0 and 7.0.

31. The use of (i) a viral surface antigen component that includes a surfactant and (ii) a fatty adjuvant, in the manufacture of a medicament for administering to a patient, wherein the weight ratio of the fatty adjuvant in (ii) to surfactant in (i) is less than 1000:1.

32. A process for preparing an immunogenic composition, comprising the steps of combining (i) an HBsAg component that includes polysorbate 20 and (ii) an adjuvant component comprising 3D-MPL adsorbed to an aluminium phosphate, to give a composition in which the weight ratio of 3D-MPL to polysorbate 20 is less than 1000:1.

33. An immunogenic composition, wherein: (a) the composition comprises HBsAg, polysorbate 20, 3D-MPL and an aluminium phosphate adjuvant wherein the polysorbate 20 is incorporated into HBsAg particles during purification; and (b) the weight ratio of the 3D-MPL to polysorbate 20 is less than 1000:1.

## Patentansprüche

1. Verfahren zur Herstellung einer immunogenen Zusammensetzung mit den Schritten des Kombinierens (i) einer viralen Oberflächenantigenkomponente, die ein Tensid enthält, und (ii) einer fetthaltigen Adjuvanskomponente unter Erhalt einer Zusammensetzung, bei der das Gewichtsverhältnis des fetthaltigen Adjuvans zu dem Tensid weniger als 1000:1 beträgt.

2. Verfahren nach Anspruch 1, wobei das fetthaltige Adjuvans ein metabolisierbares Öl umfasst.

3. Verfahren nach Anspruch 2, wobei das fetthaltige Adjuvans eine Öl-in-Wasser-Emulsion von Squalen und Polysorbat 80 im Submikrometermassstab umfasst.

4. Verfahren nach Anspruch 1, wobei das fetthaltige Adjuvans ein eine Fettsäuregruppierung umfassendes Molekül umfasst.

5. Verfahren nach Anspruch 4, wobei es sich bei dem fetthaltigen Adjuvans um ein 3-deoxyacyliertes Monophosphoryllipid A (,3D-MPL') handelt.

6. Verfahren nach Anspruch 5, wobei es sich bei dem 3D-MPL um ein Gemisch von in ihrer Acylierung variierenden Molekülen handelt.

7. Verfahren nach Anspruch 5 oder 6, wobei das fetthaltige Adjuvans Folgendes enthält:

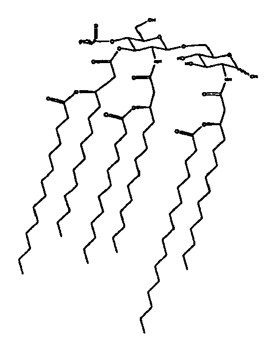

8. Verfahren nach einem der Ansprüche 5 bis 7, wobei das 3D-MPL in Form von Partikeln vorliegt.

9. Verfahren nach Anspruch 8, wobei sich die Partikel filtersterilisieren lassen.

10. Verfahren nach einem der Ansprüche 5 bis 9, wobei das 3D-MPL in Kombination mit einem Aluminiumsalz vorliegt.

11. Verfahren nach Anspruch 10, wobei es sich bei dem Aluminiumsalz um ein Aluminiumphosphat handelt.

12. Verfahren nach Anspruch 11, wobei das 3D-MPL an das Aluminiumsalz adsorbiert wird.

13. Verfahren nach einem vorhergehenden Anspruch, wobei es sich bei dem Tensid um einen Polyoxyethylensorbitanester handelt.

14. Verfahren nach Anspruch 13, wobei es sich bei dem Ester um Polysorbat 20 handelt.

15. Verfahren nach Anspruch 13 oder Anspruch 14, wobei die Tensidkonzentration nicht mehr als 50 µg/ml beträgt.

16. Verfahren nach einem vorhergehenden Anspruch, wobei es sich bei dem viralen Oberflächenantigen um ein in Gegenwart von Tensid aufgereinigtes partikuläres Hepatitis-B-Oberflächenantigen (,HBsAg') handelt.

17. Verfahren nach Anspruch 16, wobei das HBsAg in einer Hefezelle exprimiert wird.

18. Verfahren nach Anspruch 17, wobei es sich bei der Hefe um eine *Saccharomyces cerevisiae* handelt.

19. Verfahren nach einem der Ansprüche 16 bis 18, wobei das HBsAg nicht glykosyliert ist und/oder Phosphatidylinositol enthält.

20. Verfahren nach einem der Ansprüche 16 bis 19, wobei das HBsAg aus Hepatitis-B-Virus-Subtyp adw2 stammt .

21. Verfahren nach einem vorhergehenden Anspruch, wobei es sich bei dem Antigen um ein ein virales Oberflächenantigen und ein heterologes Antigen umfassendes Hybridprotein handelt.

22. Verfahren nach Anspruch 21, wobei es sich bei dem viralen Oberflächenantigen um HBsAg und bei dem heterologen Antigen um ein Malariaantigen handelt.

23. Verfahren nach Anspruch 22, wobei das Hybridprotein HBsAg und ein Fragment des CS(Circumsporozoite)-Proteins aus *Plasmodium falciparum* umfasst.

24. Verfahren nach Anspruch 23, wobei das Hybridprotein einen C-terminalen Anteil eines *P.falciparum*-CS(Circumsporozoite)-Proteins aus Plasmodium falciparum, vier oder mehr Tandem-Wiederholungen des immundominanten Bereichs von CS-Protein sowie HBsAg umfasst.

25. Verfahren nach einem vorhergehenden Anspruch, wobei das Gewichtsverhältnis des fetthaltigen Adjuvans zu dem Tensid weniger als 500:1 beträgt.

26. Verfahren nach einem vorhergehenden Anspruch, wobei das Gewichtsverhältnis des fetthaltigen Adjuvans zu dem Tensid weniger als 50:1 beträgt.

27. Verfahren nach einem vorhergehenden Anspruch, wobei es sich bei dem fetthaltigen Adjuvans um ein 3D-MPL handelt und das Gewichtsverhältnis des fetthaltigen Adjuvans zu dem Tensid zwischen 2,5:1 und 25:1 liegt.

28. Verfahren nach einem vorhergehenden Anspruch, wobei die Zusammensetzung eine Osmolalität zwischen 200 mOsm/kg und 400 mOsm/kg aufweist.

29. Verfahren nach einem vorhergehenden Anspruch, wobei die Zusammensetzung einen Phosphatpuffer enthält.

30. Verfahren nach einem vorhergehenden Anspruch, wobei die Zusammensetzung einen pH-Wert zwischen 6,0 und 7,0 aufweist.

31. verwendung (i) einer viralen Oberflächenantigenkomponente, die ein Tensid enthält, und (ii) eines fetthaltigen Adjuvans bei der Herstellung eines Arzneimittels zur Verabreichung an einen Patienten, wobei das Gewichtsverhältnis des fetthaltigen Adjuvans in (ii) zu dem Tensid in (i) weniger als 1000:1 beträgt.

32. Verfahren zur Herstellung einer immunogenen Zusammensetzung mit den Schritten des Kombinierens (i) einer HBsAg-Komponente, die Polysorbat 20 enthält, und (ii) einer Adjuvanskomponente, die an ein Aluminiumphosphat adsorbiertes 3D-MPL umfasst, unter Erhalt einer Zusammensetzung, bei der das Gewichtsverhältnis von 3D-MPL zu Polysorbat 20 weniger als 1000:1 beträgt.

33. Immunogene Zusammensetzung, wobei: (a) die Zusammensetzung HBsAg, Polysorbat 20, 3D-MPL und ein Aluminiumphosphat-Adjuvans umfasst, wobei das Polysorbat 20 während der Aufreinigung in HBsAg-Partikel eingebaut wird; und (b) das Gewichtsverhältnis des 3D-MPL zu Polysorbat 20 weniger als 1000:1 beträgt.

## Revendications

1. Procédé pour préparer une composition immunogène, comprenant les étapes de combinaison de (i) un composant d'antigène de surface viral qui comprend un tensioactif et (ii) un composant d'adjuvant gras, pour obtenir une composition dans laquelle le rapport en poids de l'adjuvant gras au tensioactif est inférieur à 1000:1.

2. Procédé de la revendication 1, dans lequel l'adjuvant gras comprend une huile métabolisable.

3. Procédé de la revendication 2, dans lequel l'adjuvant gras comprend une émulsion d'huile dans l'eau submicronique de squalène et de polysorbate 80.

4. Procédé de la revendication 1, dans lequel l'adjuvant gras comprend une molécule comprenant un fragment d'acide gras.

5. Procédé de la revendication 4, dans lequel l'adjuvant gras est un lipide A monophosphorylé 3-désoxyacylé (« 3D-MPL »).

6. Procédé de la revendication 5, dans lequel le 3D-MPL est un mélange de molécules variant en terme d'acylation.

7. Procédé de la revendication 5 ou la revendication 6, dans lequel l'adjuvant gras comprend :

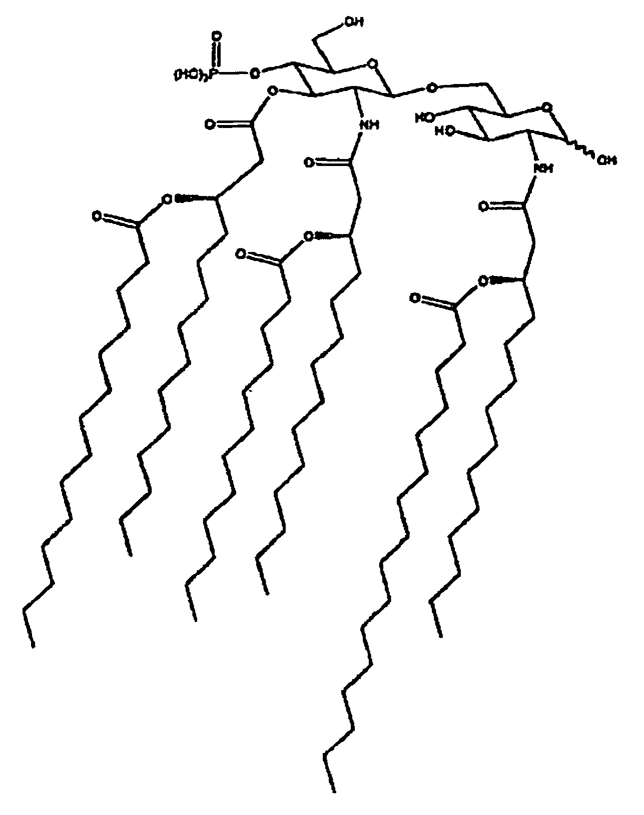

8. Procédé de l'une quelconque des revendications 5 à 7, dans lequel le 3D-MPL est sous la forme de particules.

9. Procédé de la revendication 8, dans lequel les particules peuvent être stérilisées par filtration.

10. Procédé de l'une quelconque des revendications 5 à 9, dans lequel le 3D-MPL est en combinaison avec un sel d'aluminium.

11. Procédé de la revendication 10, dans lequel le sel d'aluminium est un phosphate d'aluminium.

12. Procédé de la revendication 11, dans lequel le 3D-MPL est adsorbé sur le sel d'aluminium.

13. Procédé de l'une quelconque des revendications précédentes, dans lequel le tensioactif est un ester de polyoxyéthylène-sorbitan.

14. Procédé de la revendication 13, dans lequel l'ester est le polysorbate 20.

15. Procédé de la revendication 13 ou la revendication 14 dans lequel la concentration de tensioactif est de pas plus de 50 µg/ml.

16. Procédé de l'une quelconque des revendications précédentes, dans lequel l'antigène de surface virale est un antigène de surface d'hépatite B particulaire (« HBsAg »), purifié en présence de tensioactif.

17. Procédé de la revendication 16, dans lequel le HBsAg est exprimé dans une cellule de levure.

18. Procédé de la revendication 17, dans lequel la levure est *Saccharomyces cerevisiae*.

19. Procédé de l'une quelconque des revendications 16 à 18, dans lequel le HBsAg est non glycosylé et/ou comprend du phosphatidylinositol.

20. Procédé de l'une quelconque des revendications 16 à 19, dans lequel le HBsAg est du sous-type adw2 du virus de l'hépatite B.

21. Procédé de l'une quelconque des revendications précédentes, dans lequel l'antigène est une protéine hybride comprenant un antigène de surface virale et un antigène hétérologue.

22. Procédé de la revendication 21, dans lequel l'antigène de surface virale est HBsAg et l'antigène hétérologue est un antigène du paludisme.

23. Procédé de la revendication 22, dans lequel la protéine hybride comprend HBsAg et un fragment de la protéine circumsporozoïtaire de *Plasmodium falciparum*.

24. Procédé de la revendication 23, dans lequel la protéine hybride comprend une partie C-terminale d'une protéine circumsporozoïtaire de *P. falciparum* de Plasmodium falciparum, quatre répétitions en tandem ou plus de la région immunodominante de protéine circumsporozoïtaire, et HBsAg.

25. Procédé de l'une quelconque des revendications précédentes, dans lequel le rapport en poids de l'adjuvant gras au tensioactif est inférieur à 500:1.

26. Procédé de l'une quelconque des revendications précédentes, dans lequel le rapport en poids de l'adjuvant gras au tensioactif est inférieur à 50:1.

27. Procédé de l'une quelconque des revendications précédentes, dans lequel l'adjuvant gras est un 3D-MPL et le rapport en poids de l'adjuvant gras au tensioactif est compris entre 2,5:1 et 25:1.

28. Procédé de l'une quelconque des revendications précédentes, dans lequel la composition a une osmolalité comprise entre 200 mOsm/kg et 400 mOsm/kg.

29. Procédé de l'une quelconque des revendications précédentes, dans lequel la composition comprend un tampon phosphate.

30. Procédé de l'une quelconque des revendications précédentes, dans lequel la composition a un pH compris entre 6,0 et 7,0.

31. Utilisation de (i) un composant d'antigène de surface viral qui comprend un tensioactif et (ii) un adjuvant gras, dans la fabrication d'un médicament pour administration à un patient, où le rapport en poids de l'adjuvant gras dans (ii) au tensioactif dans (i) est inférieur à 1000:1.

32. Procédé pour préparer une composition immunogène, comprenant les étapes de combinaison de (i) un composant HBsAg qui comprend du polysorbate 20 et (ii) un composant adjuvant comprenant 3D-MPL adsorbé à un phosphate d'aluminium, pour obtenir une composition dans laquelle le rapport en poids de 3D-MPL au polysorbate 20 est inférieur à 1000:1.

33. Composition immunogène, dans laquelle : (a) la composition comprend HBsAg, polysorbate 20, 3D-MPL et un adjuvant de phosphate d'aluminium où le polysorbate 20 est incorporé dans des particules de HBsAg pendant la purification ; et (b) le rapport en poids du 3D-MPL au polysorbate 20 est inférieur à 1000:1.


### SEQUENCE LISTING

<110> NOVARTIS VACCINES AND DIAGNOSTICS SRL CONTORNI Mario
<120> REDUCING INTERFERENCE BETWEEN OIL-CONTAINING ADJUVANTS AND SURFACTANT-CONTAINING ANTIGENS
<130> PD41598W0
<140> PCT/IB2006/YYYYYY
   <141> 2006-08-02
<150> GB-0515906.6
   <151> 2005-08-02
<150> GB-0522599.0
   <151> 2005-11-04
<150> GB-0523923.1
   <151> 2005-11-24
<160> 2
<170> SeqWin99, version 1.02
<210> 1
   <211> 4
   <212> PRT
   <213> Plasmodium falciparum
<400> 1
   Asn Ala Asn Pro
   1
<210> 2
   <211> 423
   <212> PRT
   <213> Plasmodium falciparum
<400> 2
   Met Met Ala Pro Asp Pro Asn Ala Asn Pro Asn Ala Asn Pro Asn Ala
   1               5                   10                  15
   Asn Pro Asn Ala Asn Pro Asn Ala Asn Pro Asn Ala Asn Pro Asn Ala
               20                  25                  30
   Asn Pro Asn Ala Asn Pro Asn Ala Asn Pro Asn Ala Asn Pro Asn Ala
           35                  40                  45
   Asn Pro Asn Ala Asn Pro Asn Ala Asn Pro Asn Ala Asn Pro Asn Ala
       50                  55                  60
   Asn Pro Asn Ala Asn Pro Asn Ala Asn Pro Asn Ala Asn Pro Asn Ala
   65                  70                  75                  80
   Asn Pro Asn Lys Asn Asn Gln Gly Asn Gly Gln Gly His Asn Met Pro
                   85                  90                  95
   Asn Asp Pro Asn Arg Asn Val Asp Glu Asn Asn Ala Asn Asn Ala Val
               100                 105                 110
   Lys Asn Asn Asn Asn Glu Glu Pro Ser Asp Lys His Ile Glu Gln Tyr
   115 120 125
   Leu Lys Lys Ile Lys Asn Ser Ile Ser Thr Glu Trp Ser Pro Cys Ser
   130 135 140
   Val Thr Cys Gly Asn Gly Ile Gln Val Arg Ile Lys Pro Gly Ser Ala
   145 150 155 160
   Asn Lys Pro Lys Asp Glu Leu Asp Tyr Glu Asn Asp Ile Glu Lys Lys
   165 170 175
   Ile Cys Lys Met Glu Lys Cys Ser Ser Val Phe Asn Val Val Asn Ser
   180 185 190
   Arg Pro Val Thr Asn Met Glu Asn Ile Thr Ser Gly Phe Leu Gly Pro
   195 200 205
   Leu Leu Val Leu Gln Ala Gly Phe Phe Leu Leu Thr Arg Ile Leu Thr
   210 215 220
   Ile Pro Gln Ser Leu Asp Ser Trp Trp Thr Ser Leu Asn Phe Leu Gly
   225 230 235 240
   Gly Ser Pro Val Cys Leu Gly Gln Asn Ser Gln Ser Pro Thr Ser Asn
   245 250 255
   His Ser Pro Thr Ser Cys Pro Pro Ile Cys Pro Gly Tyr Arg Trp Met
   260 265 270
   Cys Leu Arg Arg Phe Ile Ile Phe Leu Phe Ile Leu Leu Leu Cys Leu
   275 280 285
   Ile Phe Leu Leu Val Leu Leu Asp Tyr Gln Gly Met Leu Pro Val Cys
   290 295 300
   Pro Leu Ile Pro Gly Ser Thr Thr Thr Asn Thr Gly Pro Cys Lys Thr
   305 310 315 320
   Cys Thr Thr Pro Ala Gln Gly Asn Ser Met Phe Pro Ser Cys Cys Cys
   325 330 335
   Thr Lys Pro Thr Asp Gly Asn Cys Thr Cys Ile Pro Ile Pro Ser Ser
   340 345 350
   Trp Ala Phe Ala Lys Tyr Leu Trp Glu Trp Ala Ser Val Arg Phe Ser
   355 360 365
   Trp Leu Ser Leu Leu Val Pro Phe Val Gln Trp Phe Val Gly Leu Ser
   370 375 380
   Pro Thr Val Trp Leu Ser Ala Ile Trp Met Met Trp Tyr Trp Gly Pro
   385 390 395 400
   Ser Leu Tyr Ser Ile Val Ser Pro Phe Ile Pro Leu Leu Pro Ile Phe
   405 410 415
   Phe Cys Leu Trp Val Tyr Ile
   420